(19) Europäisches Patentamt
European Patent Office
Office européen des brevets

(11) Veröffentlichungsnummer: **0 088 046**
**B1**

(12) # EUROPÄISCHE PATENTSCHRIFT

(45) Veröffentlichungstag der Patentschrift: **09.12.87**

(51) Int. Cl.⁴: **A 61 K 9/50**

(21) Anmeldenummer: **83810060.0**

(22) Anmeldetag: **11.02.83**

(54) **Lipide in wässriger Phase.**

(30) Priorität: **17.02.82 CH 981/82**
**17.01.83 CH 237/83**

(43) Veröffentlichungstag der Anmeldung:
**07.09.83 Patentblatt 83/36**

(45) Bekanntmachung des Hinweises auf die Patenterteilung:
**09.12.87 Patentblatt 87/50**

(84) Benannte Vertragsstaaten:
**AT BE CH DE FR GB IT LI LU NL SE**

(56) Entgegenhaltungen:
**EP - A - 0 004 223**
**EP - A - 0 055 576**
**DE - A - 2 902 672**
**FR - A - 2 344 290**
**US - A - 4 053 585**

(73) Patentinhaber: **CIBA-GEIGY AG, Klybeckstrasse 141, CH-4002 Basel (CH)**

(72) Erfinder: **Hauser, Helmut, Dr., Schwarzbachstrasse 91, CH-8713 Uerikon (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gegenstand der vorliegenden Erfindung ist ein Verfahren zur Herstellung von unilamellaren Liposomen in wässriger Phase.

Liposomen sind in der Literatur in zahlreichen Veröffentlichungen beschrieben worden. Ihr Aufbau und ihre Verwendung ist Gegenstand vieler Untersuchungen. Man unterscheidet unilamellare Liposomen mit einer Doppelschicht aus Lipiden von multilamellaren Liposomen mit mehreren Doppelschichten aus Lipiden, die zwiebelschalenförmig angeordnet sind.

Unilamellare Liposomen haben eine kugelförmige Hülle und beispielsweise einen Durchmesser von ca. $2{,}0 \times 10^{-8}$–$5{,}0 \times 10^{-6}$ m (ca. 200 bis 50 000 Å), vorzugsweise ca. $2{,}0 \times 10^{-8}$–$3{,}0 \times 10^{-6}$ m (ca. 200 bis 30 000 Å). Die kugelförmige Hülle besteht aus einer Doppelschicht der Lipidkomponenten, z.B. amphipatischen Lipiden, z.B. Phospholipiden, z.B. Phosphatidsäure, Lecithin oder Kephalin, und gegebenenfalls neutralen Lipiden, z.B. Cholesterin. Diese Doppelschicht umschliesst einen Innenraum, der eine wässrige Phase enthält. Unilamellare Liposomen werden auch als «Vesikel» bezeichnet.

Es besteht grosses Interesse an der therapeutischen Verwendung von Liposomen als Träger von Wirkstoffen unterschiedlichster Art. So sind Liposomen als Träger von Proteinen, z.B. Antikörpern oder Enzymen, Hormonen, Vitaminen oder Genen oder zu analytischen Zwecken als Träger von markierten Verbindungen vorgeschlagen worden. Als Beispiel sei die US-Patentschrift 3 993 754 genannt, welche ein chemotherapeutisches Verfahren bei der Behandlung von Tumorzellen unter Verwendung von Liposomen als Träger zum Gegenstand hat.

Der betreffende Wirkstoff wird entweder bei der Bildung der Liposomen oder nachträglich durch Diffusion verkapselt. Die Herstellung von Liposomen und die Verkapselung des Wirkstoffs kann auf verschiedene Weise erfolgen und ist in dem Übersichtsartikel «Liposomes-Problems and promise as selective drug carriers» von Kaye, St. B., Cancer Treatment Reviews (1981) 8, 27–50, beschrieben. Weitere Verfahren zur Herstellung von Liposomen zwecks Verkapselung von Wirkstoffen sind ebenfalls durch Barenholz et al. in Biochemistry, Vol 16, No. 12, 2806–2810, sowie in den Deutschen Offenlegungsschriften (DOS) 28 19 855, 29 02 672, 25 32 317 und 28 42 608, in der US-Patentschrift 4 053 585 und in der Europäischen Patentanmeldung 36 676 beschrieben.

Man löst beispielsweise die Lipidkomponenten, z.B. Phospholipide, z.B. Phosphatidsäure, Lecithin oder Kephalin, und gegebenenfalls neutrale Lipide, z.B. Cholesterin, in einem organischen Lösungsmittel, z.B. Chloroform oder Benzol, auf. Nach dem Eindampfen bleibt eine homogene Schicht, z.B. eine Filmschicht, der betreffenden Lipidkomponenten zurück. Man dispergiert anschliessend die Lipidkomponenten in einer wässrigen Phase, welche den betreffenden Wirkstoff enthält, z.B. durch Schütteln. Bei der anschliessenden Behandlung mit Ultraschall bilden sich unilamellare Liposomen, welche den Wirkstoff verkapseln.

Nach vielen bisher bekannt gewordenen Verfahren erhält man wässrige Phasen sowohl mit Mischungen von unilamellaren als auch multilamellaren Liposomen, wobei Struktur und Grösse dieser Liposomen zufällig und kaum beeinflussbar sind und beträchtlich variieren können. Wässrige Phase mit überwiegendem Anteil an unilamellaren Liposomen erhält man bisher nur mit apparativ aufwendigen Herstellungsverfahren, z.B. durch Ultraschallbehandlung, Dialysieren oder Gelfiltration.

Nach dem Verfahren der vorliegenden Erfindung lassen sich auf einfache Weise wässrige Phasen mit einem hohen bis fast quantitativen Anteil an unilamellaren Liposomen herstellen, welche kleine unilamellare Liposomen (KUL) mit einem Durchmesser von ca. $2{,}0$–$6{,}0 \times 10^{-8}$ m (ca. 200–600 Å) und grosse unilamellare Liposomen (GUL) mit einem Durchmesser von ca. $6{,}0 \times 10^{-8}$ m–$3{,}0 \times 10^{-7}$ m ca. (600–3000 Å) enthalten können. Ein besonderer Vorteil des erfindungsgemässen Verfahrens besteht darin, dass man KUL und GUL von relativ einheitlicher Grösse erhält und dass man das Mengenverhältnis von KUL zu GUL in der dispersen Phase variieren kann. Mittels geeigneter Trennmethoden, z.B. Gelfiltration oder einer Ultrafiltrationszelle, kann man kleine von grossen unilamellaren Liposomen abtrennen.

Die vorliegende Erfindung betrifft ein Verfahren zur Herstellung von unilamellaren Liposomen, dadurch gekennzeichnet, dass man

a) ein Lipid der Formel

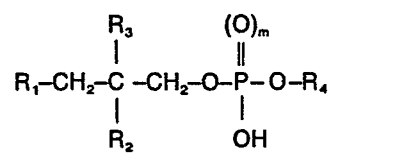

(A),

worin m null oder eins ist, einer der Reste $R_1$ und $R_2$ Wasserstoff, Hydroxy, $C_1$–$C_4$-Alkyl und der andere Rest Alkyl, Alkenyl, Alkoxy oder Alkenyloxy mit je 10–20 C-Atomen oder Acyloxy mit 10–50 C-Atomen, $R_3$ Wasserstoff oder $C_1$–$C_4$-Alkyl und $R_4$ Wasserstoff, $C_1$–$C_4$-Alkyl, $C_1$–$C_7$-Alkyl substituiert durch Carboxy oder Sulfo, $C_1$–$C_7$-Alkyl substituiert durch Carboxy und Amino, wobei die Aminogruppe sich in α-Stellung zur Carboxygruppe befindet, $C_1$–$C_4$-Alkyl substituiert durch Halogen, $C_1$–$C_4$-Alkoxycarbonyl oder $C_1$–$C_4$-Alkansulfonyl oder $C_2$–$C_4$-Alkyl substituiert durch freie oder verätherte Hydroxygruppen, wobei zwei verätherte Hydroxygruppen durch Methylen, Äthylen, Äthyliden, 1,2-Propylen oder 2,2-Propylen miteinander verbunden sein können, einen Kohlehydratrest mit 5–12 C-Atomen oder, wenn $R_1$ und $R_2$ Wasserstoff oder Hydroxy und $R_3$ Wasserstoff bedeuten, einen Steroidrest bedeuten, und ein zusätzliches Lipid der Formel A, worin m null oder eins ist, $R_1$ und $R_2$ unabhängig voneinander Alkyl, Alkenyl, Alkoxy oder Alkenyloxy mit je 10–20 C-Atomen, $R_3$ Wasserstoff und $R_4$ durch Tri-$C_1$–$C_4$-alkylammonio sub-

stituiertes $C_2$–$C_4$-Alkyl oder 2-Aminoäthyl bedeuten, oder dass man ein Lipid der Formel A, worin m null oder eins ist, $R_1$ und $R_2$ unabhängig voneinander Alkyl, Alkenyl, Alkoxy mit je 10–20 C-Atomen oder Acyloxy mit 10–50 C-Atomen, $R_3$ und $R_4$ Wasserstoff bedeuten und gegebenenfalls eines der genannten zusätzlichen Lipide der Formel A in wässriger Phase mit einem pH-Wert grösser als neun dispergiert, oder dass man

b) ein Lipid der Formel A, worin m null oder eins ist, einer der Reste $R_1$ und $R_2$ Wasserstoff, Hydroxy, $C_1$–$C_4$-Alkyl und der andere Rest Alkyl, Alkenyl, Alkoxy oder Alkenyloxy mit je 10–20 C-Atomen oder Acyloxy mit 10–50 C-Atomen, $R_3$ Wasserstoff und $R_4$ durch eine Ammoniogruppe substituiertes $C_2$–$C_4$-Alkyl bedeuten, und gegebenenfalls eins der unter a) genannten zusätzlichen Lipide der Formel A oder dass man ein Lipid der Formel A, worin m null oder eins ist, $R_1$ und $R_2$ unabhängig voneinander Alkyl, Alkenyl oder Alkenyl oder Alkenyloxy mit je 10–20 C-Atomen oder Acyloxy mit 10–50 C-Atomen, $R_3$ Wasserstoff und $R_4$ durch eine Ammonio-$C_2$–$C_4$-alkylammoniogruppe substituiertes $C_2$–$C_4$-Alkyl bedeuten, und eins der unter a) genannten zusätzlichen Lipide der Formel A in wässriger Phase mit einem pH-Wert bis ca. 1 oder darunter dispergiert und die wässrige Phase neutralisiert und, wenn erwünscht, die erhältlichen unilamellaren Liposomen anreichert und/oder abtrennt.

Die weiter vorn und im folgenden verwendeten allgemeinen Begriffe haben im Rahmen der vorliegenden Beschreibung vorzugsweise die folgenden Bedeutungen:

Verfahren a)

Alkyl $R_1$, $R_2$ oder $R_3$ mit 1–4 C-Atomen ist z.B. bevorzugt Methyl, ferner Äthyl, n-Propyl, oder n-Butyl.

Alkyl $R_1$ oder $R_2$ ist vorzugsweise n-Decyl, n-Undecyl, n-Dodecyl (Lauryl), n-Tridecyl, n-Tetradecyl (Myristyl), n-Pentadecyl, n-Hexadecyl (Cetyl), n-Octadecyl (Stearyl) oder n-Eicosyl (Arachinyl), ferner n-Heptadecyl oder n-Nonadecyl.

Alkenyl $R_1$ oder $R_2$ ist vorzugsweise
9-cis-Dodecenyl (Lauroleyl),
9-cis-Tetradecenyl (Myristoleyl),
9-cis-Hexadecenyl (Palmitoleinyl),
6-cis-Octadecenyl (Petroselinyl),
6-trans-Octadecenyl (Petroselaidinyl),
9-cis-Octadecenyl (Oleyl),
9-trans-Octadecenyl (Elaidinyl) oder
9-cis-Eicosenyl (Gadoleinyl),
ferner 1-Decenyl, 1-Undecenyl, 1-Dodecenyl,
1-Tridecenyl, 1-Tetradecenyl,
1-Pentadecenyl,
1-Hexadecenyl, 1-Heptadecenyl, 1-Octadecenyl,
9-cis-12-trans-Octadecadienyl (Linolyl),
9-trans-12-trans-Octadecadienyl (Linolaidinyl),
9-cis-12-cis-Octadienyl (Linoleyl),
9-cis-11-trans-13-trans-Octadecatrienyl (β-Eläostearinyl),
9-cis-12-cis-15-cis-Octadecatrienyl (Linolenyl),
9-, 11-, 13-, 15-Octadecatetraenyl (Parinaryl),
1-Nonadecencyl, 1-Eicosenyl,
5-, 11-, 14-Eicosatrienyl oder 5-, 8-, 11-, 14-Eicosatetraenyl (Arachidonyl).

Alkoxy $R_1$ oder $R_2$ ist vorzugsweise n-Decyloxy, n-Dodecyloxy (Lauryloxy), n-Tetradecyloxy (Myristyloxy), n-Hexadecyloxy (Cetyloxy), n-Octadecyloxy (Stearyloxy) oder n-Eicosyloxy (Arachinyloxy), ferner n-Undecyloxy, n-Tridecyloxy, n-Pentadecyloxy, n-Heptadecyloxy oder n-Nonadecyloxy.

Alkenyloxy $R_1$ oder $R_2$ ist vorzugsweise
9-cis-Dodecenyloxy (Lauroleyloxy),
9-cis-Tetradecenyloxy (Myristoleyloxy),
9-cis-Hexadecenyloxy (Palmitoleinyloxy),
6-cis-Octadecenyloxy (Petroselinyloxy),
6-trans-Octadecenyloxy (Petroselaidinyloxy),
9-cis-Octadecenyloxy (Oleyloxy),
9-trans-Octadecenyloxy (Elaidinyloxy) oder
9-cis-Eicosenyl (Gadoleinyloxy),
ferner 1-Decenyloxy, 1-Undecenyloxy,
1-Dodecenyloxy, 1-Tridecenyloxy,
1-Tetradecenyloxy, 1-Pentadecenyloxy,
1-Hexadecenyloxy, 1-Heptadecenyloxy,
1-Octadecenyloxy,
9-cis-12-trans-Octadecadienyloxy (Linolyloxy),
9-trans-12-trans-Octadecadienyloxy (Linolaidinyloxy),
9-cis-12-cis-Octadienyloxy (Linoleyloxy),
9-cis-11-trans-13-trans-Octadecatrienyloxy (β-Eläostearinyloxy),
9-cis-12-cis-15-cis-Octadecatrienyloxy (Linolenyloxy),
9-, 11-, 13-, 15-Octadecatetraenyloxy (Parinaryloxy),
1-Nonadecenyloxy, 1-Eicosenyloxy,
5-, 11-, 14-Eicosatrienyloxy oder 5-, 8-, 11-, 14-Eicosatetraenyloxy (Arachidonyloxy).

Acyloxy $R_1$ oder $R_2$ mit je 10–50 C-Atomen ist beispielsweise Alkanoyloxy, durch ein aromatisches Ringsystem substituiertes Alkanoyloxy oder Alkenoyloxy.

Alkanoyloxy $R_1$ oder $R_2$ ist vorzugsweise
n-Decanoyloxy, n-Dodecanoyloxy (Lauroyloxy),
n-Tetradecanoyloxy (Myristoyloxy),
n-Hexadecanoyloxy,
n-Hexadecanoyloxy (Palmitoyloxy),
n-Octadecanoyloxy (Stearoyloxy) oder
n-Eicosoyloxy (Arachinoyloxy),
ferner n-Undecanoyloxy, n-Tridecanoyloxy,
n-Pentadecanoyloxy, n-Heptadecanoyloxy oder
n-Nonadecanoyloxy.

Durch ein aromatisches Ringsystem substituiertes Alkanoyloxy $R_1$ oder $R_2$ ist beispielsweise Phenyl-n-alkanoyloxy, worin der Phenylrest sich in ω-Stellung des Alkanoyloxyrests befindet, z.B.
Phenyl-n-butyryloxy, -n-pentanoyloxy,
-n-hexanoyloxy, -n-heptanoyloxy,
-n-octanoyloxy, -n-nonanoyloxy, -n-decanoyloxy,
-n-undecanoyloxy oder
Phenyl-n-dodecanoyloxy,
3- oder 4-, vorzugsweise 4-Alkylphenyl-n-alkanoyloxy,
worin der Alkylphenylrest sich in ω-Stellung des Alkanoyloxyrests befindet, z.B.
4-n-Butyl-, 4-n-Pentyl-, 4-n-Hexyl-, 4-n-Octyl-,

4-n-Decyl- oder 4-n-Dodecylphenyl-n-butyryloxy,
-n-pentanoyloxy-, n-hexanoyloxy,
-n-octanoyloxy, -n-decanoyloxy oder
-n-dodecanoyloxy,
Pyren-1-yl-n-alkanoyloxy,
worin der Pyrenrest sich in ω-Stellung des Alkanoyloxyrests befindet, z.B.
Pyren-1-yl-n-butyryloxy, -n-pentanoyloxy,
-n-hexanoyloxy, -n-octanoyloxy,
-n-decanoyloxy oder
Pyren-1-yl-decanoyloxy, oder
6- oder 8-Alkylpyren-1-yl-n-alkanoyloxy,
worin der Alkylpyren-1-ylrest sich in ω-Stellung des Alkanoyloxyrests befindet, z.B. 6- oder 8-Niederalkyl-, z.B.
6- oder 8-Äthylpyren-1-yl-n-butyryloxy,
-n-pentanoyloxy, -n-hexanoyloxy,
-n-octanoyloxy, -n-decanoyloxy oder
-n-decanoyloxy, oder
6- oder 8-n-Butylpyren-1-yl-n-butyryloxy,
-n-pentanoyloxy, -n-hexanoyloxy,
-n-octanoyloxy, n-decanoyloxy oder
-n-dodecanoyloxy, oder
6- oder 8-Alkylpyren-1-yl-n-alkanoyloxy, z.B.
6- oder 8-n-Decyl-, -n-Dodecyl-, -n-Tetradecyl,
-n-Hexadecyl- oder
6- oder 8-n-Octadecylpyren-1-yl-n-butyryloxy,
-n-pentanoyloxy, n-hexanoyloxy, n-octanoyloxy,
-n-decanoyloxy oder -n-dodecanoyloxy.

Durch ein aromatisches Ringsystem substituiertes Alkanoyloxy $R_1$ oder $R_2$ ist vorzugsweise
4-(4-n-Decylphenyl)-decanoyl,
4-(Pyren-1-yl)-butanoyl,
6-(Pyren-1-yl)-hexanoyl,
8-(Pyren-1-yl)-octanoyl,
10-(Pyren-1-yl)-octanoyl,
6-(6- oder 8-Äthylpyren-1-yl)-octanoyl,
6-(6- oder 8-n-Butylpyren-1-yl)-hexanoyl und
10-(6- oder 8-n-Octadecylpyren-1-yl)-decanoyl.

Alkenoyloxy $R_1$ oder $R_2$ ist vorzugsweise
9-cis-Dodecenyloxy (Lauroleoyloxy),
9-cis-Tetradecenoyloxy (Myristoleoyloxy),
9-cis-Hexadecenoyloxy (Palmitoleinoyloxy),
6-cis-Octadecenoyloxy (Petroselinoyloxy),
6-trans-Octadecenoyloxy (Petroselaidinoyloxy),
9-cis-Octadecenoyloxy (Oleoyloxy),
9-trans-Octadecenoyloxy (Elaidinoyloxy) oder
9-cis-Eicosenoyl (Gadoleinoyloxy),
ferner 9-cis-12-trans-Octadienoyloxy (Linoloyl),
9-trans-12-trans-Octadecadienoyloxy
(Linolaidinoyloxy),
9-cis-12-cis-Octadienoyloxy (Linoleoyloxy),
9-cis-11-trans-13-trans-Octadecatrienoyloxy
(Linolenoyloxy),
9-, 11-, 13-, 15-Octadecatetraenoyloxy
(Parinaroyloxy),
5-, 11-, 14-Eicosatrienoyloxy oder 5-, 8-,
11-, 14-Eicosatetraenoyloxy (Arachidonoyloxy).

$R_4$ ist beispielsweise Wasserstoff, $C_1$–$C_4$-Alkyl, z.B. Methyl, Äthyl, Isopropyl, n-Propyl, Isobutyl oder n-Butyl, $C_1$–$C_7$-Alkyl substituiert durch Carboxy oder Sulfo, $C_1$–$C_7$-Alkyl substituiert durch Carboxy und Amino, wobei die Aminogruppe sich in α-Stellung zur Carboxygruppe befindet, $C_1$–$C_4$-Alkoxycarbonyl oder $C_1$–$C_4$-Alkansulfonyl oder ist $C_2$–$C_4$-Alkyl substituiert durch freie oder verätherte Hydroxygruppen, wobei zwei verätherte Hydroxygruppen durch Methylen, Äthylen, Äthyliden, 1,2-Propylen oder 2,2-Propylen miteinander verbunden sein können.

$C_1$–$C_7$-Alkyl substituiert durch Carboxy oder Sulfo $R_4$ ist vorzugsweise Carboxymethyl, 2-Carboxyäthyl oder 3-Carboxy-n-propyl. $C_1$–$C_7$-Alkyl substituiert durch Carboxy und Amino, worin die Aminogruppe sich in α-Stellung zur Carboxygruppe befindet, ist beispielsweise 2-Amino-2-carboxyäthyl oder 3-Amino-3-carboxy-n-propyl.

$C_1$–$C_4$-Alkyl substituiert durch Halogen ist beispielsweise Chlor- oder Brommethyl, 2-Chlor- oder 2-Bromäthyl oder 2- oder 3-Chlor- oder 2- oder 3-Brom-n-propyl.

$C_2$–$C_4$-Alkyl substituiert durch freie oder verätherte Hydroxygruppen, wobei zwei verätherte Hydroxygruppen durch Methylen, Äthylen, Äthyliden, 1,2-Propylen oder 2,2-Propylen miteinander verbunden sein können, ist beispielsweise 2,3-(2,2-Propylen)-dioxypropyl oder 2,3-Äthylendioxypropyl.

$R_4$ ist ebenfalls ein Kohlehydratrest mit 5–12 C-Atomen, beispielsweise ein natürlicher Monosaccharidrest, der sich von einer als Aldose oder Ketose vorliegenden Pentose oder Hexose ableitet.

Eine als Aldose vorliegende Pentose ist z.B. D-Ribose, D-Arabinose, D-Xylose oder D-Lyxose.

Eine als Ketose vorliegende Pentose ist z.B. D-Ribulose oder D-Xylulose.

Eine als Aldose vorliegende Hexose ist z.B. D-Allose, D-Altrose, D-Glucose, D-Mannose, D-Galactose oder D-Talose.

Eine als Ketose vorliegende Hexose ist z.B. D-Psicose, D-Fructose, D-Sorbose oder D-Tagatose.

Eine Hexose liegt vorzugsweise in zyklischer Form vor, z.B. als Pyranose (Aldose), z.B. α- oder β-D-Glucopyranose, oder als Furanose, z.B. α- oder β-D-Fructofuranose. Der Pyranosylrest ist vorzugsweise durch die in 1- oder 6-Stellung und der Furanosylrest durch in 1- oder 5-Stellung befindliche Hydroxygruppe mit der Phosphatidylgruppe verestert.

Ein Kohlehydratrest $R_4$ mit 5–12 C-Atomen ist ferner ein natürlicher Disaccharidrest, z.B. ein aus zwei Hexosen gebildeter Disaccharidrest, der beispielsweise durch Kondensation von zwei Aldosen, z.B. D-Glucose oder D-Galactose oder einer Aldose, z.B. D-Glucose mit einer Ketose, z.B. Fructose, gebildet wird. Aus zwei Aldosen gebildete Disaccharide, z.B. Lactose oder Maltose, sind vorzugsweise über die in 6-Stellung des betreffenden Pyranosylrests befindliche Hydroxygruppe mit der Phosphatidylgruppe verestert. Aus einer Aldose und einer Ketose gebildete Disaccharide, z.B. Saccharose, sind vorzugsweise über die in 6-Stellung des Pyranosylrests oder über die in 1-Stellung des Furanosylrests bedindliche Hydroxygruppe mit der Phosphatidylgruppe verestert.

Ein Kohlehydratrest $R_4$ mit 5–12 C-Atomen ist ferner ein derivatisierter Mono- oder Disaccharidrest, worin beispielsweise die Aldehydgruppe und/oder ein oder zwei endständige Hydroxygruppen zu Carboxylgruppen oxydiert sind, z.B. ein D-Glucon-, D-Glucar- oder D-Glucoronsäurerest, welche vorzugsweise als zyklische Lactonreste vorliegen. Ebenso können in einem derivatisierten Mono- oder Disaccharidrest Aldehyd- oder Ketogruppen zu Hydroxygruppen reduziert sein, z.B. Inosit, Sorbit oder D-Mannit, oder Hydroxygruppen durch Wasserstoff, z.B. Desoxyzucker, z.B. 2-Desoxy-D-ribose, L-Rhamnose oder L-Fucose, oder durch Aminogruppen, z.B. Aminozucker, z.B. D-Glucosamin oder D-Galactosamin, ersetzt sein.

Ein Kohlehydrat $R_4$ kann ebenfalls ein durch Umsetzung eines der genannten Mono- oder Disaccharide mit einem starken Oxydationsmittel, z.B. Peryodsäure, gebildetes Spaltprodukt sein.

Wenn R und $R_2$ Wasserstoff oder Hydroxy und $R_3$ Wasserstoff bedeuten, kann $R_4$ einen Steroidrest darstellen, z.B. einen Sterinrest, der über die in 3-Stellung des Steroidgerüsts befindliche Hydroxygruppe mit der Phosphatidylgruppe verestert ist.

Ein Sterinrest ist beispielsweise Lanosterin, Sitosterin, Koprostanol, Chloestanol, Glykocholsäure, Ergosterin oder Stigmasterin, vorzugsweise Cholesterin.

Wenn $R_4$ einen Steroidrest darstellt, sind $R_1$ und $R_2$ vorzugsweise Hydroxy und $R_3$ ist Wasserstoff.

Ein geeignetes zusätzliches Lipid ist beispielsweise ein Lipid der Formel A, worin m null oder eins ist, $R_1$ und $R_2$ unabhängig voneinander Alkyl, Alkenyl, Alkoxy oder Alkenyloxy mit je 10–20 C-Atomen oder Acyloxy mit 10–50 C-Atomen mit den weiter vorn genannten Bedeutungen, $R_3$ Wasserstoff und $R_4$ durch Triniederalkylammonio substituiertes $C_2$–$C_4$-Alkyl, z.B. 2-Trimethylammonioäthyl (Cholinyl) oder 2-Aminoäthyl, darstellen.

Ein solches zusätzliches Lipid ist z.B. ein natürliches Lecithin, z.B. Ei-Lecithin oder Lecithin aus Sojabohnen, wenn $R_4$ 2-Trimethylammonioäthyl bedeutet, und ein natürliches Kephalin, z.B. Ei-Kephalin oder Kephalin aus Sojabohnen, wenn $R_4$ 2-Aminoäthyl bedeutet.

Ausserdem sind als zusätzliche Lipide synthetische Lecithine ($R_4$ = 2-Trimethylammonioäthyl) und synthetische Kephaline ($R_4$ = 2-Aminoäthyl) der Formel A bevorzugt, worin $R_1$ und $R_2$ identische Acyloxyreste, z.B.
Lauroyloxy, Oleoyloxy, Linoyloxy, Linoleoyloxy oder Arachinoyloxy bedeuten, z.B.
Dilauroyl-, Dimyristoyl-, Dipalmitoyl-,
Distearoyl-, Diarachinoyl-, Dioleoyl-,
Dilinoyl-, Dilinoleoyl-, oder
Diarachinoyllecithin oder -kephalin,
$R_1$ und $R_2$ verschiedene Acyloxyreste, z.B. $R_1$ Palmitoyloxy und $R_2$ Oleoyloxy, z.B. 1-Palmitoyl-2-oleoyl-lecithin oder -kephalin, $R_1$ und $R_2$ identische Alkoxyreste, z.B. Tetradecyloxy oder Hexadecyloxy, z.B. Ditetradecyl- oder Dihexadecyllecithin oder -kephalin, $R_1$ Alkenyl und $R_2$ Acyloxy, z.B. ein Plasmalogen ($R_4$ = Trimethylammonioäthyl), oder $R_1$ Acyloxy, z.B. Myristoyloxy oder Palmitoyloxy, und $R_2$ Hydroxy, z.B. ein natürliches oder synthetisches Lysolecithin oder Lysokephalin, z.B. 1-Myristoyl- oder 1-Palmitoyllysolecithin oder -kephalin, und $R_3$ Wasserstoff darstellen.

Ein geeignetes zusätzliches Lipid ist ferner ein Lipid der Formel A, worin m eins ist, $R_1$ Alkenyl, $R_2$ Acylamido, $R_3$ Wasserstoff und $R_4$ einen 2-Trimethylammonioäthyl-Rest (Cholinrest) darstellen. Ein solches Lipid ist unter dem Namen Sphingomyelin bekannt.

Bevorzugt enthält die wässrige Dispersion ein Lipid der Formel A, worin m eins ist, $R_1$ Alkyl, z.B.
n-Dodecyl (Lauryl), n-Tridecyl,
n-Tetradecyl (Myristyl), n-Pentacedyl,
n-Hexadecyl (Cetyl), n-Heptadecyl oder
n-Octadecyl (Stearyl), Alkoxy, z.B.
n-Dodecyloxy (Lauryloxy),
n-Tetradecyloxy (Myristyloxy),
n-Hexadecyloxy (Cetyloxy), oder
n-Octadecyloxy (Stearyloxy), Acyloxy, z.B.
Lauroyloxy, Myristoyloxy, Palmitoyloxy oder Stearoyloxy,
$R_2$ Wasserstoff oder Hydroxy, $R_3$ Wasserstoff oder Niederalkyl, z.B. Methyl, und $R_4$ Wasserstoff, Niederalkyl, z.B. Methyl oder Äthyl, 2-Amino-2-carboxyäthyl oder
3-Amino-3-carboxy-n-propyl, 2-Hydroxyäthyl,
2,3-Äthylendioxypropyl oder
2,3-(2,2-Propylen)-dioxypropyl,
Halogen-$C_2$–$C_4$-alkyl, z.B.
2-Chlor- oder 2-Bromäthyl,
oder einen Kohlenhydratrest mit 5–12 C-Atomen, z.B. Inosit, bedeuten, und ein zusätzliches Lipid der Formel A, worin $R_1$ und $R_2$ Acyloxy, z.B. Lauroyloxy, Myristoyloxy, Palmitoyloxy oder Stearoyloxy, $R_3$ Wasserstoff und $R_4$ 2-Trimethylammonioäthyl oder 2-Aminoäthyl bedeuten. Die wässrige Dispersion kann auch bevorzugt ein Lipid der Formel A, worin $R_1$ und $R_2$ Acyloxy, z.B. Lauroyloxy, Myristoyloxy, Palmitoyloxy oder Stearoyloxy, $R_3$ Wasserstoff und $R_4$ Wasserstoff bedeuten, und gegebenenfalls ein zusätzliches Lipid der Formel A, worin $R_1$ und $R_2$ Acyloxy, z.B. Lauroyloxy, Myristoyloxy, Palmitoyloxy oder Stearoyloxy, $R_3$ Wasserstoff und $R_4$ 2-Trimethylammonioäthyl, 2-Aminoäthyl, Niederalkyl substituiert durch saure und basische Gruppen, z.B. Carboxy und Amino, z.B. ω-Amino-ω-carboxyniederalkyl, z.B. 2-Amino-2-carboxyäthyl oder 3-Amino-3-carboxy-n-propyl, oder einen Kohlehydratrest mit 5–12 C-Atomen bedeuten, z.B. Inosit, enthalten.

In erster Linie enthält die wässrige Dispersion natürliche Lysophosphatidsäure, z.B. Ei-Lysophosphatidsäure, oder eine synthetische Lysophosphatidsäure, z.B. 1-Lauroyl-, 1-Myristoyl- oder 1-Palmitoyllysophosphatidsäure, natürliches Lysophosphatidylserin, z.B. Lysophosphatidylserin aus dem Rinderhirn, oder synthetisches Lysophosphatidylserin, z.B. 1-Myristoyl- oder 1-Palmitoyllysophosphatidylserin, ein Lysophosphatidylglycerin oder ein Lysophosphatidylinositol und zusätzlich natürliches Lecithin, z.B. Ei-Lecithin, Lecithin mit gleichen Acyloxygruppen, z.B. Dimyristoyl- oder Dipalmitoyllecithin, ein Lecithin mit verschiedenen Acyloxygruppen, z.B. 1-Palmitoyl-2-oleoyllecithin, oder zusätzlich natürliches

Kephalin, z.B. Ei-Kephalin, oder ein Kephalin mit verschiedenen Acyloxygruppen, z.B. 1-Palmitoyl-2-oleoylkephalin.

In erster Linie kann die wässrige Dispersion auch eine natürliche Phosphatidsäure, z.B. Ei-Phosphatidsäure, eine synthetische Phosphatidsäure, z.B. Dilauroyl-, Dimyristoyl-, Dipalmitoyl- oder 1-Palmitoyl-2-oleoylphosphatidsäure, und gegebenenfalls zusätzlich natürliches Lecithin, z.B. Ei-Lecithin, ein Lecithin mit gleichen Acyloxygruppen, z.B. Dimyristoyl- oder Dipalmitoyllecithin, oder ein Lecithin mit verschiedenen Acyloxygruppen, z.B. 1-Palmitoyl-2-oleoyllecithin, oder natürliches Kephalin, z.B. Ei-Kephalin oder ein Kephalin mit verschiedenen Acyloxygruppen, z.B. 1-Palmitoyl-2-oleoylkephalin, oder ein Phosphatidylserin, z.B. ein natürliches Phosphatidylserin, z.B. Phosphatidylserin aus dem Rinderhirn, oder ein synthetisches Phosphatidylserin, z.B. Dipalmitoylphosphatidylserin, ein Monoglycerid, z.B. Monoolein oder Monomyristin, oder ein Sterin, z.B. Cholesterin, enthalten.

Zur Herstellung von unilamellaren Liposomen stellt man zunächst eine homogene Schicht der Lipidkomponenten her. Die Herstellung der homogenen Schicht kann in an sich bekannter Weise erfolgen und ist weiter hinten im Abschnitt «Herstellung der homogenen Schicht der Lipidkomponenten» beschrieben.

Die homogene Schicht dispergiert man in wässriger Phase und erhöht anschliessend den pH-Wert von solchen wässrigen Phasen, worin nur eine Lipidkomponente, z.B. reine Phosphatidsäure, dispergiert ist, bis auf ca. 12, bevorzugt bis auf ca. 9 bis 11. Dies erfolgt beispielsweise durch Zugabe von physiologisch annehmbaren, basischen Lösungen, z.B. verdünnter wässriger, ca. 0,01–0,2 N, insbesondere ca. 0,1 N Natriumhydroxid- oder Kaliumhydroxid-Lösung, unter gleichzeitiger Kontrolle des pH-Werts. z.B. durch Tüpfelprobe oder ein pH-Meter.

In einer bevorzugten Ausführungsform dispergiert man die homogene Schicht der Lipidkomponenten in wässrigen Phasen mit einem pH-Wert grösser als 11, z.B. in physiologisch annehmbaren, basischen Lösungen, z.B. in verdünnter wässriger, ca. 0,01–0,2 N, insbesondere 0,1 N Natriumhydroxid- oder Kaliumhydroxid-Lösung. Eine Lipidkomponente, z.B. reine Phosphatidsäure, dispergiert man in wässrigen Phasen mit einem pH-Wert bis ca. 12, bevorzugt ca. 9 bis 11.

Verfahren b)

Für ein Lipid der Formel A, worin m null oder eins ist, einer der Reste $R_1$ und $R_2$ Wasserstoff, Hydroxy, Niederalkyl mit 1–4 C-Atomen und der andere Rest Alkyl, Alkenyl, Alkoxy oder Alkenyloxy mit je 10–20 C-Atomen oder Acyloxy mit 10–50 C-Atomen, $R_3$ Wasserstoff und $R_4$ durch eine Ammoniogruppe substituiertes Niederalkyl bedeuten, haben $R_1$ und $R_2$ die weiter vorn unter Verfahren a) genannten Bedeutungen.

Durch eine Ammoniogruppe substituiertes Niederalkyl $R_4$ ist beispielsweise durch eine Triniederalkylammoniogruppe, z.B. Trimethyl- oder Triäthylammonio, substituiertes Niederalkyl, z.B. 2-Trimethyl- oder 2-Triäthylammonioäthyl.

Für ein Lipid der Formel A, worin m null oder eins ist, $R_1$ und $R_2$ unabhängig voneinander Alkyl, Alkenyl, Alkoxy oder Alkenyloxy mit je 10–20 C-Atomen oder Acyloxy mit 10–50 C-Atomen, $R_3$ Wasserstoff und $R_4$ durch eine Ammonioniederalkylammoniogruppe substituiertes Niederalkyl bedeuten, haben $R_1$ und $R_2$ die weiter vorn unter Verfahren a) genannten Bedeutungen.

Durch eine Ammonioniederalkylammoniogruppe substituiertes Niederalkyl ist beispielsweise
2-[N,N-Diniederalkyl-N-(2-N',N',N'-triniederalkylammonioäthyl)-ammonio]-äthyl, z.B.
2-[N,N-Dimethyl-N-(2-N',N',N'-trimethylammonioäthyl)-ammonio]-äthyl.

Ein geeignetes zusätzliches Lipid ist eins der weiter vorn unter Verfahren a) genannten zusätzlichen Lipide.

Bevorzugt enthält die wässrige Dispersion ein Lipid der Formel A, worin m eins ist, $R_1$ Acyloxy, z.B. Lauroyloxy, Myristoyloxy, Palmitoyloxy oder Stearoyloxy, $R_2$ Hydroxy, $R_3$ Wasserstoff und $R_4$ 2-Trimethylammonioäthyl bedeuten, und ein zusätzliches Lipid der Formel A, worin $R_1$ und $R_2$ Acyloxy, z.B. Lauroyloxy, Myristoyloxy, Palmitoyloxy oder Stearoyloxy, $R_3$ Wasserstoff und $R_4$ 2-Aminoäthyl oder 2-Trimethylammonioäthyl bedeuten. Die wässrige Dispersion kann auch bevorzugt ein Lipid der Formel A, worin $R_1$ und $R_2$ Acyloxy, z.B. Lauroyloxy, Myristoyloxy, Palmitoyloxy oder Stearoyloxy, $R_3$ Wasserstoff und $R_4$
2-[N,N-Dimethyl-N-(2-N',N',N'-trimethylammonioäthyl)-ammonio]-äthyl
bedeuten und gegebenenfalls ein zusätzliches Lipid der Formel A, worin $R_1$ und $R_2$ Acyloxy, z.B. Lauroyloxy, Myristoyloxy, Palmitoyloxy oder Stearoyloxy, $R_3$ Wasserstoff und $R_4$ 2-Aminoäthyl oder 2-Trimethylammonioäthyl bedeuten, enthalten.

In erster Linie enthält die wässrige Dispersion ein Lysophosphatidylcholin (Lysolecithin) und ein natürliches Lecithin, z.B. Ei-Lecithin. In erster Linie kann die wässrige Dispersion auch ein
Phosphatidyl-2-[N,N-Dimethyl-N-(2-N',N',N'-trimethylammonioäthyl)-ammonio]-äthylchlorid
und gegebenenfalls ein natürliches Lecithin, z.B. Ei-Lecithin, enthalten.

Zur Herstellung von unilamellaren Liposomen stellt man zunächst eine homogene Schicht der Lipidkomponenten, z.B. Lysolecithin oder
Phosphatidyl-2-[N,N-Dimethyl-N-(2-N',N',N'-trimethylammonioäthyl)-ammonio]-äthylchlorid,
her.

Die Herstellung der homogenen Schicht kann in an sich bekannter Weise erfolgen und ist weiter hinten im Abschnitt «Herstellung der homogenen Schicht der Lipidkomponenten» beschrieben.

Die homogene Schicht dispergiert man in wässriger Phase und erniedrigt anschliessend den pH-Wert bis auf ca. 1 oder darunter unter gleichzeitiger Kontrolle des pH-Werts, z.B. durch Tüpfelproben oder ein pH-Meter. Dies erfolgt beispielsweise durch Zugabe von physiologisch annehmbaren Säuren, beispielsweise verdünnten wässrigen Mineralsäuren, z. B. verdünnter wässriger Schwefelsäure, Salzsäure oder Phosphorsäure.

In einer bevorzugten Ausführungsform dispergiert man die homogene Schicht der Lipidkomponenten in wässrigen Phasen mit einem pH-Wert von ca. 1 oder Werten darunter, z. B. in verdünnten wässrigen Mineralsäuren, z. B. verdünnter wässriger Schwefelsäure, Salzsäure oder Phosphorsäure unter gleichzeitiger Kontrolle des pH-Werts.

Eine anschliessende Neutralisierung der wässrigen Phasen ist notwendig, wenn man zuvor den pH-Wert der wässrigen Phase gemäss Verfahren a) auf Werte höher als 9 oder gemäss Verfahren b) niedriger als 1 eingestellt hat. Dies erfolgt, um unmittelbar nach der pH-Wert Erniedrigung oder Erhöhung eine Zerstörung des Wirkstoffs und/oder der Liposomen unter basischen bzw. sauren Bedingungen zu vermeiden. Die basisch gemachte wässrige Phase neutralisiert man mit einer geeigneten physiologisch annehmbaren Säure oder einer Pufferlösung, z. B. Phosphatpufferlösung mit einem pH-Wert von 7 bis 8. Geeignete Säuren sind beispielsweise die weiter vorn genannten verdünnten wässrigen Mineralsäuren sowie schwache organische Säuren, z. B. Ameisensäure oder Essigsäure. Die saure wässrige Phase neutralisiert man durch Zugabe von wässrigen Basen, z. B. verdünnter wässriger Natrium- oder Kaliumhydroxid-Lösung. Man neutralisiert unter gleichzeitiger Kontrolle des pH-Werts.

Die Lipide sind in Konzentrationen bis über 70% in der wässrigen Phase dispergiert. Der Konzentrationsbereich von ca. 1% bis ca. 20% ist bevorzugt.

Man arbeitet zweckmässigerweise bei Raumtemperatur oder höheren Temperaturen, z. B. bis ca. 60 °C. Falls es die Empfindlichkeit des zu verkapselnden Werkstoffs verlangt, führt man das Verfahren unter Kühlen und gegebenenfalls in einer Inertgasatmosphäre, z. B. Stickstoffatmosphäre, durch.

Sowohl nach Verfahren a) als auch nach Verfahren b) findet die Bildung von unilamellaren Liposomen spontan (spontaneous vesiculation), d. h. ohne zusätzliche Energiezufuhr von aussen und mit grosser Geschwindigkeit, statt.

Die nach Verfahren a) und b) erhältlichen unilamellaren Liposomen sind in wässriger Phase relativ lange stabil. Beispielsweise bleiben unilamellare Liposomen bestehend aus Ei-Phosphatidsäure oder Ei-Phosphatidsäure und Ei-Lecithin in wässeriger Phase bei 4 °C gelagert mehr als 14 Tage lang stabil. Wässrige Phasen mit erfindungsgemäss herstellbaren unilamellaren Liposomen können nach den in der Europäischen Patentanmeldung 0 065 292 angegebenen Verfahren lagerungsfähig gemacht werden.

Die erfolgte Bildung von unilamellaren Liposomen und ihr Gehalt in wässriger Phase lassen sich in an sich bekannter Weise anhand verschiedener Messmethoden, z. B. optisch im Elektronenmikroskop, durch Massenbestimmung in der analytischen Ultrazentrifuge und vor allem spektroskopisch, z. B. im Kernresonanzspektrum ($^{1}H$, $^{13}C$ und $^{31}P$), nachweisen. So geben beispielsweise scharfe Signale im Kernresonanzspektrum einen Hinweis auf erfolgte Bildung von kleinen unilamellaren Liposomen. Der Anteil an gebildeten kleinen unilamellaren Liposomen im System kann aus der Intensität der Signale berechnet werden. So ist im Protonen-Kernresonanzspektrum ein scharfes Methylensignal bei $\delta = 1{,}28$ ppm und ein scharfes Methylsignal bei $\delta = 0{,}89$ ppm für kleine unilamellare Liposomen, welche aus Phosphatidsäure gebildet werden, charakteristisch. Kleine unilamellare Liposomen, welche aus Phosphatidsäure und Lecithin bestehen, zeigen ebenfalls das Methylen- und das Methylsignal bei $\delta = 1{,}28$ und 0,89 ppm und zusätzlich ein Methylsignal bei $\delta = 3{,}23$ ppm, welches der Trimethylammoniogruppe des Lecithins zugeordnet wird.

Die Grösse der gebildeten unilamellaren Liposomen ist u. a. von der Struktur der Lipidkomponenten, dem Mischungsverhältnis der Lipidkomponenten, der Konzentration dieser Lipidkomponenten in der wässrigen Phase und von der Menge und Struktur des zu verkapselnden Wirkstoffs abhängig. So kann man beispielsweise durch Variation der Konzentration der Lipidkomponenten wässrige Phasen mit einem hohen Anteil an kleinen oder grossen unilamellaren Liposomen herstellen. Beispielsweise wird durch Zugabe von Ei-Phosphatidsäure zur dispersen Phase der Anteil an kleinen unilamellaren Liposomen (KUL) erhöht. Der Anteil von GUL in einer dispersen Phase lässt sich auch durch Zusatz von Salzen, z. B. NaCl oder KCl erhöhen. Der Durchmesser der beispielsweise aus Phosphatidsäure oder Phosphatidsäure und Lecithin gebildeten KUL beträgt ca. $2{,}0 \times 10^{-8}$–$6{,}0 \times 10^{-8}$ m (200–600 Å). Das Einschlussvolumen für KUL von dieser Grösse beträgt ca. 0,5 bis 1 l pro Mol eingesetzter Lipidkomponente.

Zusätzlich zu KUL enstehen auch grosse unilamellare Liposomen (GUL-Durchmesser bis zu $5.0 \times 10^{-6}$ m (50 000 Å). Diese schliessen grössere Volumina pro Mol eingesetzter Lipidkomponenten ein und eignen sich zur Verkapselung mit höherer Ausbeute und zum Einschluss von voluminösen Materialien, z. B. Viren, Bakterien oder Zellorganellen.

Die Trennung der KUL von GUL erfolgt mittels herkömmlicher Trennmethoden, z. B. Gelfiltration, z. B. mit Sepharose 4B als Träger, oder durch Sedimentation der GUL in der Ultrazentrifuge bei 160 000 × g. Beispielsweise setzen sich nach mehrstündigem, ca. dreistündigem, Zentrifugieren in diesem Schwerefeld die GUL ab, während die KUL dispergiert bleiben und dekantiert werden können. Nach mehrmaligem Zentrifugieren erreicht man eine vollständige Trennung der GUL von KUL.

Auch durch Gelfiltration kann man alle in der wässrigen Phase befindlichen Liposomen mit einem Durchmesser grösser als $6{,}0 \times 10^{-8}$ m (600Å), z. B. GUL oder multilamellare Liposomen, sowie nicht verkapselte Wirkstoffe und überschüssige, dispergierte Lipide abtrennen und so eine wässrige Phase mit einer Fraktion KUL von relativ einheitlicher Grösser erhalten.

Die erfindungsgemäss erhältlichen Liposomen (KUL und GUL) sind geeignete Trägersysteme, welche in wässriger Phase zur Solubilisierung von lipophilen Stoffen, z.B. fettlöslichen Farbstoffen, zur Stabilisierung von hydrolyseempfindlichen Stoffen, z.B. Prostaglandinen, zum Einschluss von Schädlingsbekämpfungsmitteln, z.B. zur Veränderung des Wirkungsprofils von Dichlorphos, zum Einschluss von Nahrungsmittelzusätzen, z.B. zwecks Änderung des Adsorptionsverhaltens von Vitaminen oder Farbstoffen, oder zur Einschleusung von verkapselten Wirkstoffen, Enzymen, Antikörpern, Hormonen, Genen, Viren, Vitaminen oder Zellorganellen in die Zellen einer Zellkultur verwendet werden können.

Wässrige Phasen, welche die erfindungsgemäss erhältlichen Liposome mit verkapselten Wirkstoffen enthalten, sind Verabreichungssysteme, welche sich, gegebenenfalls nach Konzentrierung oder Isolierung der Liposomen, z.B. durch Ultrazentrifugieren, zu therapeutischen Zwecken für die orale (p.o.), parenterale (i.v., i.m. oder i.p.) oder topikale Verabreichung eignen.

Bei oraler Verabreichung können Verabreichungssysteme auf Liposomenbasis einen Wirkstoff, beispielsweise Insulin, das im Verdauungstrakt unbeständig ist, schützen oder seine Resorption verbessern. Für die orale Verabreichung kann die Liposomen-haltige wässrige Phase mit pharmazeutisch unbedenklichen Verdünnungsmitteln oder Trägern oder mit üblichen Zusätzen, z.B. Farbstoffen oder Geschmacksstoffen, vermischt und als Sirup oder in Form von Kapseln verabreicht werden.

Bei parenteraler Verabreichung können Verabreichungssysteme auf Liposomenbasis beispielsweise die Verweilzeit z.B. von Desferrioxamin, siehe Guilmette R.A. et al., Life Sci. 22 (4) 313–320, 1978, oder Gentamycin, siehe Scheld W.M. et al., Clin. Res. 26, No. 1, 59 A, 1978, in einem Organismus verlängern. Ebenso wird die Verweilzeit von verkapselten Chelatbildern, z.B. EDTA (Äthylendiamintetraessigsäure), in Organismen verlängert, so dass man durch Chelatbildung Schwermetalle besonders aus Leber, Milz oder Nieren entfernen kann, siehe Rahmann et al., Science, Vol. 180, 300–302, 1973, und J. Lab. Clin. Med. 640–647, 1974. Mit Verabreichungssystemen auf Liposomenbasis kann man Wirkstoffe im Myokard anreichern, siehe Landesmann et al., Science Vol. 198, 737–738, 1977. Antiinflammatorisch wirkende Stoffe, z.B. Cortisol, siehe Nature 271, No. 5643, 372–73, 1978, oder Proteaseinhibitoren, siehe Anal. Biochem. 89, No. 2, 400–07, 1978, kann man in der Gelenkflüssigkeit und Cytostatika in Tumorgewebe, siehe Übersichtsartikel von Kaye St. B., Cancer Treatment Reviews 8, 27–50, 1981, und die vielen darin zitierten Literaturstellen, anreichern. Manche Chemotherapeutika in der Krebstherapie sind weniger toxisch und besser verträglich, wenn sie in Liposomen verkapselt verabreicht werden, z.B. liposomverkapseltes Actinomycin D. siehe Rahman et al., Proceedings of the Society for Experimental Biology and Medicine 146, 1173–1176, 1974, Methotrexat, siehe Lesermann L.D. et al. Proc. Natl. Acad. Sci. 77, No 7, 4089–93, 1980, Vinblastin, Daunomycin oder Cytosin-Arabinosid, siehe Mühlensiepen et al., Cancer Res. 41, Nr. 5, 1602–07, 1981. Liposomen können zur Einschleusung von Wirkstoffen, z.B. Enzymen, Peptidhormonen, Genen oder Viren in das Cytoplasma von Zellen in lebenden Organismen, z.B. zur Einschleusung von Asparaginase, siehe Übersichtsartikel von Finkelstein M. und Weissmann G., J. Lipid Research, Vol. 19, 1978, 289–303, von Amyloglucosidase, siehe Gregoriadis G. und Ryman B.E., Eur. J. Biochem. 24 (1972), 485–491, oder Neurominidase, siehe Gregoriadis et al., Biochem. J. (1974) 140, 232–330, zur Verankerung spezifischer Erkennungsmoleküle, z.B. monoklonaler Antikörper, zwecks zielgerichteter Einschleusung in definierte Zielzellen, siehe Leserman et al., Nature 293 (5829), 226–228, 1981, zur Immunstimulation als Adjuvans bei Impfungen, z.B. gegen Leishmaniasen, siehe New R.R.C. et al. Nature 272 (5648) 55–56, 1978, oder zur induzierten Freisetzung von Wirkstoffen durch Signale wie Temperaturerhöhungen, z.B. in entzündetem Gewebe, oder pH-Wert Änderungen verwendet werden. Für die parenterale Verabreichung können die konzentrierten oder isolierten Liposomen in einer geeigneten Trägerflüssigkeit, beispielsweise in sterilem destilliertem Wasser oder in physiologischer Kochsalzlösung, suspendiert werden.

Herstellung der homogenen Schicht der Lipidkomponenten

Die Herstellung der homogenen Schicht der Lipidkomponenten kann in an sich bekannter Weise erfolgen. Beispielsweise löst man zunächst das Lipid oder Lipidgemisch der Formel A, z.B. reine Ei-Phosphatidsäure oder eine Mischung aus Ei-Phosphatidsäure und Ei-Lecithin, gegebenenfalls unter Zumischung eines lipophilen Wirkstoffs, z.B. Proteins, das bei der Bildung der Liposomen in der Lipidschicht, eingeschlossen wird, in einem organischen Lösungsmittel auf. Durch Entfernen des organischen Lösungsmittels, am zweckmässigsten im Vakuum oder durch Abblasen im Inertgas, z.B. Stickstoff, stellt man eine homogene Schicht der Lipidkomponenten her.

Die Auswahl des betreffenden Lösungsmittels ist von der Löslichkeit der betreffenden Lipidkomponenten darin abhängig. Geeignete Lösungsmittel sind beispielsweise unsubstituierte oder substituierte, z.B. halogenierte, aliphatische, cycloaliphatische, aromatische oder aromatisch-aliphatische Kohlenwasserstoffe, z.B. Benzol, Toluol, Methylenchlorid oder Chloroform, Alkohole, z.B. Methanol oder Äthanol, Niederalkancarbonsäureester, z.B. Essigsäureäthylester, Äther, z.B. Diäthyläther, Dioxan oder Tetrahydrofuran, oder Mischungen dieser Lösungsmittel.

Die in der Beschreibung der vorliegenden Erfindung erwähnten Lipide sind bekannt oder können, falls sie neu sind, in an sich bekannter Weise nach den im Standardwerk von Knight C.G. Liposomes,

Elsevier 1981, Kapitel 3, befindlichen Vorschriften hergestellt werden. Alle genannten Lipide können in der wässrigen Dispersion als optisch aktive Derivate oder als Racemate enthalten sein. Die folgenden Beispiele veranschaulichen die Erfindung, ohne sie zu beschränken. Temperaturen sind in Grad Celsius angegeben und Mischungsverhältnisse auf Volumina bezogen.

Beispiel 1:

a) Man löst 1 g Ei-Phosphatidsäure in 20 ml einer Chloroform/Methanol Mischung (2:1) und dampft diese Lösung im Vakuum im Rotationsverdampfer ein. Man dispergiert den filmartigen Rückstand in 20 ml destilliertem Wasser durch fünf Minuten langes Schütteln, wobei sich ein pH-Wert von ca. 3 einstellt. Zur Bildung von unilamellaren Liposomen gibt man anschliessend zur dispersen Phase bei Raumtemperatur unter Kontrolle mit einem pH-Meter soviel 0,1 N Natriumhydroxid-Lösung, bis der pH-Wert auf 11 steigt. Der pH-Wert der wässrigen Phase wird anschliessend durch Zugabe von 0,1 N HCl von 11 bis auf ca. 7 gesenkt. Man erhält eine leicht opaleszierende, wässrige Phase.

Die gebildeten unilamellaren Liposomen können im Elektronenmikroskop sichtbar gemacht werden. Die Liposomendispersion wird zunächst der üblichen Gefrierbruchmethode (freeze-fracture) unterzogen. Es liegen hauptsächlich zwei «Populationen» von unilamellaren Liposomen vor, die sich durch ihre durchschnittliche Grösse unterscheiden:

1. Kleine unilamellare Liposomen (KUL) mit einem Durchmesser von ca. $2{,}0 \times 10^{-8}$–$6{,}0 \times 10^{-8}$ m (200–600 Å) und

2. Grosse unilamellare Liposomen (GUL) mit einem Durchmesser von ca. $1{,}0 \times 10^{-7}$–$1{,}0 \times 10^{-6}$ m (1 000–10 000 Å).

KUL sind im Protonen-NMR-Spektrum durch die Signale $\delta = 1{,}28$ (Methylen) und $\delta = 0{,}89$ ppm (Methyl) erkennbar. Die Ausbeute an KUL kann aus den Intensitäten der Signale abgeschätzt werden und beträgt ca. 56%.

b) Analog Beispiel 1a) löst man 4 mal je 10 mg Ei-Phosphatidsäure in 4 mal je 0,2 ml einer Chloroform/Methanol Mischung (2:1) und dampft diese Lösungen im Vakuum ein. Man dispergiert jeweils den filmartigen Rückstand in 1 ml destilliertem Wasser durch 5 Minuten langes Schütteln. Zur Bildung von unilamellaren Liposomen gibt man anschliessend zu jeder einzelnen dispersen Phase unter Kontrolle mit einem pH-Meter soviel 0,1 N Natriumhydroxid-Lösung bis ein pH-Endwert von jeweils 6, 8, 11,3 und 11,6 resultiert. Die Ausbeute an KUL beträgt mit ansteigendem pH-Wert für jede Probe 5, 24, 57 und 60%.

Beispiel 2:

a) Man löst 1 g Ei-Phosphatidsäure in 20 ml einer Chloroform/Methanol Mischung (2:1) und dampft diese Lösung im Vakuum ein. Man dispergiert den filmartigen Rückstand in 50 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt ca. 100%.

b) Analog Beispiel 2a) löst man 4 mal je 10 mg Ei-Phosphatidsäure in 4 mal je 0,2 ml einer Chloroform/Methanol Mischung (2:1) und dampft diese Lösungen im Vakuum ein. Man dispergiert jede Probe in soviel 0,01 N Natriumhydroxid-Lösung und destilliertem Wasser unter Schütteln, dass sich pH-Werte von ca. 7,3, 8,0, 9,4 und 10,0 einstellen. Die Ausbeute an KUL beträgt mit ansteigendem pH-Wert für jede Probe 33, 46, 65 und 81%.

Beispiel 3:

Man löst 0,1 g Dilauroylphosphatidsäure in 5 ml einer Chloroform/Methanol Mischung (2:1) und dampft diese Lösung im Vakuum ein. Man dispergiert den filmartigen Rückstand in 50 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL ca $3{,}0 \times 10^{-8}$–$8{,}0 \times 10^{-8}$ m (300–800 Å) beträgt 73%.

Beispiel 4:

a) Man löst 3 mg Ei-Phosphatidsäure und 7 mg Ei-Lecithin in 0,5 ml einer Chloroform/Methanol Mischung (2:1) und dampft diese Lösung im Vakuum ein. Man dispergiert den filmartigen Rückstand in 1 ml destilliertem Wasser durch fünf Minuten langes Schütteln bei Raumtemperatur, worauf sich ein pH-Wert von ca. 3 einstellt. Zur Bildung von unilamellaren Liposomen gibt man anschliessend bei Raumtemperatur unter Kontrolle mit einem pH-Meter soviel 0,1 Natronlauge, bis der pH-Wert auf ca. 11,2 steigt. Durch Zugabe von Phosphatpufferlösung stellt man anschliessend den pH-Wert der wässrigen Phase auf ca. 7 ein. Man erhält eine leicht opaleszierende, wässrige Phase. Die erfolgte Bildung von unilamellaren Liposomen ist im NMR-Spektrum durch die Signale $\delta = 1{,}28$ (Methylen), $\delta = 0{,}89$ (Methyl) und $\delta = 3{,}23$ ($N$–$CH_3$) erkennbar. In der elektronenmikroskopischen Abbildung sind hauptsächlich zwei «Populationen» von unilamellaren Liposomen zu erkennen, die sich durch ihre durchschnittliche Grösse unterscheiden:

1. KUL mit einem Durchmesser von ca. $2{,}0 \times 10^{-8}$–$80 \times 10^{-8}$ m (ca. 200–800 Å) und

2. GUL mit einem Durchmesser von ca. $1{,}0 \times 10^{-7}$–$1{,}0 \times 10^{-6}$ m (ca. 1000–10 000 Å).

Die Ausbeute an KUL beträgt 45%.

b) Analog Beispiel 4a) löst man 2 mal je 3 mg Ei-Phosphatidsäure und 7 mg Ei-Lecithin in 2 mal je 0,5 ml einer Chloroform/Methanol Mischung (2:1) und dampft diese Lösungen im Vakuum ein. Man dispergiert jeweils den filmartigen Rückstand in 1,0 ml destilliertem Wasser durch 5 Minuten langes Schütteln. Zur Bildung von unilamellaren Liposomen gibt man anschliessend zu jeder einzelnen Phase unter Kontrolle mit einem pH-Meter soviel 0,1 N Natriumhydroxid-Lösung unter Schütteln, bis ein Endwert von 8,6 und 10 eingestellt ist.

Die Ausbeute an KUL beträgt mit ansteigendem pH-Wert 22 und 35%.

c) Analog Beispiel 4a) löst man Proben unterschiedlichen Gehalts an Ei-Phosphatidsäure und Ei-Lecithin in je 0,5 ml einer Choroform/Methanol Mischung (2:1) und dampft diese Lösungen im Vakuum ein. Man dispergiert jeweils den filmartigen Rückstand in 1,0 ml destilliertem Wasser durch 5 Minuten langes Schütteln. Zur Bildung von unilamellaren Liposomen gibt man anschliessend zu jeder einzelnen Phase unter Kontrolle mit einem pH-Meter soviel 0,1 N Natriumhydroxid-Lösung unter Schütteln, bis sich ein pH-Wert von ca. 11,2 eingestellt hat. Die Ausbeute an KUL beträgt für jede Probe mit steigendem Ei-Phosphatidsäuregehalt:

| | | | | | | | | | | |
|---|---|---|---|---|---|---|---|---|---|---|
| %-Ei-Phosphatidsäure | 6 | 10 | 14 | 20 | 25 | 30 | 33 | 50 | 48 | 60 |
| %-KUL | 5 | 9 | 14 | 17 | 19 | 20 | 27 | 39 | 41 | 50 |

Beispiel 5:

a) Man löst 0,3 g Ei-Phosphatidsäure und 0,7 g Ei-Lecithin in 10 ml einer Chloroform/Methanol Mischung (2:1) und dampft diese Lösung im Vakuum ein. Man dispergiert diesen Rückstand in 10 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt ca. 30%.

b) Analog Beispiel 5a) löst man Proben unterschiedlichen Gehalts an Ei-Phosphatidlösung und Ei-Lecithin (insgesamt 10 mg Lipid) in je 0,5 ml einer Chloroform/Methanol Mischung (2:1) und dampft diese Lösung im Vakuum ein. Man dispergiert jeweils den filmartigen Rückstand in je 1 ml einer 0,01 N Natriumhydroxid-Lösung unter Schütteln, wobei sich ein pH-Wert von ca. 12 einstellt. Der pH-Wert der wässrigen Dispersion wird auf ca. 7 bis 8 gesenkt. Die Ausbeute an KUL beträgt für jede Probe mit steigendem Ei-Phosphatidsäuregehalt:

| | | | | | | | | |
|---|---|---|---|---|---|---|---|---|
| %-Ei-Phosphatidsäure | 10 | 20 | 25 | 30 | 40 | 50 | 60 | 80 |
| %-KUL | 14 | 22 | 31 | 42 | 45 | 50 | 78 | 95 |

Beispiel 6:

a) Man löst 0,7 g Ei-Lecithin, 0,3 g Phosphatidylserin aus dem Rinderhirn und 2 g Ei-Phosphatidsäure in 20 ml einer Chloroform/Methanol Mischung (2:1) und dampft diese Lösung im Vakuum im Rotationsverdampfer ein. Man dispergiert den filmartigen Rückstand in 100 ml 0,01 N Natriumhydroxid-Lösung durch fünf Minuten langes Schütteln bei Raumtemperatur, wobei sich ein pH-Wert von ca. 12 einstellt. Durch Zugabe von 1 N Salzsäure wird der pH-Wert der wässrigen Phase auf ca. 7 eingestellt. Man erhält eine leicht opaleszierende, wässrige Phase.

Die erfolgte Bildung von unilamellaren Liposomen kann analog Beispiel 1a) spektroskopisch, z.B. im NMR-Spektrum, oder im Elektronenmikroskop nachgewiesen werden. In der elektronenmikroskopischen Abbildung sind GUL und KUL zu erkennen.

b) Analog Beispiel 6a) löst man Proben unterschiedlichen Gehalts an Ei-Phosphatidsäure, aber gleicher Menge an Ei-Lecithin und Phosphatidylserin (insgesamt 10 mg Lipid) in je 0,5 ml einer Chloroform/Methanol Mischung (2:1) und dampft diese Lösungen im Vakuum ein. Man dispergiert jeweils den Rückstand in je 1,0 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt für jede Probe mit steigendem Ei-Phosphatidsäuregehalt:

| | | | | | | | |
|---|---|---|---|---|---|---|---|
| %-Ei-Phosphatidsäure | 9 | 10 | 26 | 33 | 34 | 40 | 60 |
| %-KUL | 14 | 18 | 26 | 36 | 47 | 43 | 64 |

Beispiel 7:

a) Man löst 1 g Asolectin (Phospholipidgemisch hauptsächlich bestehend aus Lecithin, Kephalin, Phosphatidylserin und Phosphatidylinosit) und 0,2 g Ei-Phosphatidsäure in 20 ml einer Chloroform/Methanol Mischung (2:1) und dampft diese Lösung im Vakuum ein. Man dispergiert den filmartigen Rückstand in 100 ml 0,01 N Natriumhydroxid-Lösung durch fünf Minuten langes Schütteln bei Raumtemperatur, wobei sich ein pH-Wert von ca. 12 einstellt. Durch Zugabe von 1 N Salzsäure wird der pH-Wert der wässrigen Phase auf ca. 7 gebracht. Man erhält eine leicht opaleszierende, wässrige Phase.

Die erfolgte Bildung von unilamellaren Liposomen kann analog Beispiel 1a) spektroskopisch, z.B. im NMR-Spektrum, oder im Elektronenmikroskop nachgewiesen werden. In der elektronenmikroskopischen Abbildung sind GUL und KUL zu erkennen.

b) Analog zu Beispiel 6a) löst man Proben unterschiedlichen Gehalts an Ei-Phosphatidsäure, aber gleicher Menge an Asolectin (insgesamt 10 mg Lipid) in je 0,5 ml einer Chloroform/Methanol Mischung (2:1) und dampft diese Lösungen im Vakuum ein. Man dispergiert jeweils den Rückstand in je 1 ml einer 0,01 N Natriumhydroxid-Lösung unter Schütteln, wobei sich ein pH-Wert

von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt für jede Probe mit steigendem Ei-Phosphatidsäuregehalt:

| | | | |
|---|---|---|---|
| %-Ei-Phosphatidsäure | 17 | 37 | 50 |
| %-KUL | 24 | 69 | 65 |

Beispiel 8:

a) Man löst 0,1 g einer Mischung aus Ei-Lecithin und Cholesterin (Molverhältnis 1:1) und 0,1 g Ei-Phosphatidsäure in 10 ml einer Chloroform/Methanol Mischung (2:1) und dampft diese Lösung im Vakuum ein. Man dispergiert den filmartigen Rückstand in 10 ml 0,01 N Natriumhydroxid-Lösung durch fünf Minuten langes Schütteln bei Raumtemperatur, wobei sich ein pH-Wert von ca. 12 einstellt. Durch Zugabe von 1 N Salzsäure wird der pH-Wert der wässrigen Phase auf ca. 7 gebracht. Man erhält eine leicht opaleszierende, wässrige Phase.

Die erfolgte Bildung von unilamellaren Liposomen kann analog Beispiel 1a) spektroskopisch, z.B. im NMR-Spektrum, oder im Elektronenmikroskop, nachgewiesen werden. In der elektronenmikroskopischen Abbildung sind GUL und KUL zu erkennen.

b) Analog Beispiel 8a) löst man Proben unterschiedlichen Gehalts an Ei-Phosphatidsäure, aber gleicher Menge an Ei-Lecithin und Cholesterin (insgesamt 10 mg Lipid) in je 0,5 ml einer Chloroform/Methanol Mischung (2:1) und dampft diese Lösungen im Vakuum ein. Man dispergiert jeweils den Rückstand in je 1,0 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt für jede Probe mit steigendem Ei-Phosphatidsäuregehalt:

| | | | | |
|---|---|---|---|---|
| %-Ei-Phosphatidsäure | 10 | 30 | 50 | 80 |
| %-KUL | 4 | 10 | 20 | 50 |

Beispiel 9:

Man löst 0,5 g Ei-Phosphatidsäure und 0,5 g Dimyristoyllecithin in 10 ml einer Chloroform/Methanol Mischung (2:1) und dampft diese Lösung im Vakuum ein. Man dispergiert den filmartigen Rückstand in 50 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt 36%.

Beispiel 10:

Man stellt analog Beispiel 9 Liposomengemische bestehend aus 0,5 g Ei-Phosphatidsäure und jeweils 0,5 g Dipalmitoyllecithin oder Distearoyllecithin her. Die Ausbeute an KUL beträgt 10%.

Beispiel 11:

Man stellt analog Beispiel 9 ein Liposomengemisch bestehend aus 0,5 g Dipalmitoylphosphatidsäure und 0,5 g Ei-Lecithin her. Die Ausbeute an KUL beträgt 10%.

Beispiel 12:

Man löst 5 mg Lysolecithin und 5 mg Ei-Lecithin in 1 ml einer Chloroform/Methanol Mischung (2:1) und dampft diese Lösung im Vakuum ein. Man dispergiert den filmartigen Rückstand in 1 ml destilliertem Wasser durch fünf Minuten langes Schütteln, wobei sich ein pH-Wert von ca. 5–7 einstellt. Zur Bildung von unilamellaren Liposomen gibt man anschliessend zur wässrigen Dispersion bei Raumtemperatur unter Kontrolle mit einem pH-Meter soviel 0,1 N Salzsäure, bis der pH-Wert der wässrigen Phase auf 0,5 abgesunken ist. Durch Zugabe von 0,1 N Natriumhydroxidlösung erhöht man anschliessend den pH-Wert auf 7.

Die erfolgte Bildung von unilamellaren Liposomen kann analog Beispiel 1a) spektroskopisch, z.B. im NMR-Spektrum, oder im Elektronenmikroskop nachgewiesen werden. In der elektronenmikroskopischen Abbildung sind KUL und GUL zu erkennen. Die Ausbeute an KUL beträgt 50%.

Beispiel 13:

Man stellt analog Beispiel 12 ein Liposomengemisch bestehend aus 5 mg
Phosphatidyl-2-[N,N-dimethyl-N-(2-N',N',N'-trimethylammonioäthyl)-ammonio]-äthylchlorid,
dessen Herstellung in Knight C.G., Liposomes, Elsevier 1981, Kapitel 3, beschrieben ist, und 5 mg Ei-Lecithin her.

Die erfolgte Bildung von unilamellaren Liposomen kann analog Beispiel 1a) spektroskopisch, z.B. im NMR-Spektrum, oder im Elektronenmikroskop nachgewiesen werden. In der elektronenmikroskopischen Abbildung sind KUL mit einem Durchmesser von 250 Å und GUL mit einem Durchmesser von ca. $6{,}0 \times 10^{-8}$–>$1{,}0 \times 10^{-6}$ m (ca. 600–>10 000 Å) zu erkennen. Die Ausbeute an KUL beträgt 50%.

Beispiel 14:

a) Man löst 0,5 g Monoolein (9-cis-Octadecenoylglycerol) und 0,5 g Ei-Phosphatidsäure in 20 ml einer Chloroform/Methanol Mischung (2:1) und dampft diese Lösung im Vakuum ein. Man dispergiert den filmartigen Rückstand in 100 ml einer 0,01 N Natriumhydroxid-Lösung durch fünf Minuten langes Schütteln bei Raumtemperatur, wobei sich ein pH-Wert von ca. 12 einstellt. Durch Zugabe von 1 N Salzsäure wird der pH-Wert der wässrigen Phase auf ca. 7 gebracht. Man erhält eine leicht opaleszierende, wässrige Phase.

Die erfolgte Bildung von unilamellaren Liposomen kann analog Beispiel 1a) spektroskopisch, z.B. im NMR-Spektrum, oder im Elektronenmikroskop nachgewiesen werden. In der elektronenmikroskopischen Abbildung sind GUL und KUL zu erkennen.

b) Analog Beispiel 4a) löst man Proben unterschiedlichen Gehalts an Ei-Phosphatidsäure und an Monoolein (totale Lipidmenge = 10 mg) in je 0,5 ml einer Chloroform/Methanol Mischung (2:1) und dampft diese Lösungen im Vakuum ein. Man dispergiert jeweils den Rückstand in je 1 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt für jede Probe mit steigendem Ei-Phosphatidsäuregehalt:

| | | | | |
|---|---|---|---|---|
| %-Ei-Phosphatidsäure | 20 | 30 | 50 | 80 |
| %-KUL | 10 | 17 | 26 | 45 |

Beispiel 15:

Analog Beispiel 14a) löst man Proben unterschiedlichen Gehalts an Ei-Phosphatidsäure und Monomyristin (gesamte Lipidmenge: 10 mg) in je 0,5 ml einer Chloroform/Methanol Mischung (2:1) und dampft diese Lösungen im Vakuum ein. Man dispergiert jeweils den Rückstand in je 1 ml einer 0,01 N Natriumhydroxid-Lösung unter Schütteln, wobei sich ein pH-Wert von ca. 12 einstellt. Der pH-Wert der wässrigen Dispersion wird anschliessend durch Zugabe von 0,1 N Salzsäure auf ca. 7 bis 8 gesenkt. Die Ausbeute an KUL beträgt für jede Probe mit steigendem Ei-Phosphatidsäuregehalt:

| | | | |
|---|---|---|---|
| %-Ei-Phosphatidsäure | 30 | 50 | 80 |
| %-KUL | 9 | 18 | 38 |

Beispiel 16:

a) Man stellt analog Beispiel 1a) und 1b) ein Liposomengemisch bestehend aus Ei-Phosphatidsäure her, wobei man eine Ausbeute bis zu 66% KUL erhält. Um den Anteil an GUL im Liposomengemisch zu erhöhen, setzt man zur dispersen Phase, welche frisch hergestellt unilamellare Liposomen enthält, 0,5 molare Kochsalzlösung hinzu. Mit zunehmender NaCl-Konzentration in der dispersen Phase sinkt der Anteil an KUL:

| | | | | | | | | |
|---|---|---|---|---|---|---|---|---|
| [NaCl] in Mol/l | 0 | 0,2 | 0,3 | 0,4 | 0,5 | 0,6 | 0,75 | 0,95 |
| %-KUL | 66 | 64 | 50 | 40 | 30 | 23 | 15 | 11 |

b) Um den Anteil an GUL im Liposomengemisch zu erhöhen, setzt man zur dispersen Phase, welche frisch hergestellte unilamellare Liposomen bestehend aus reiner Ei-Phosphatidsäure enthält, 0,5 molare Kaliumchloridlösung hinzu. Mit zunehmender KCl-Konzentration in der dispersen Phase sinkt der Anteil an KUL:

| | | | | | |
|---|---|---|---|---|---|
| [KCl] in Mol/l | 0 | 0,2 | 0,4 | 0,5 | 0,63 |
| %-KUL | 66 | 63 | 50 | 50 | 36 |

Beispiel 17:

Analog Beispiel 1–16 kann man unilamellare Liposomen aus

Myristinsäure und Ei-Lecithin,
Myristinsäure und Ei-Kephalin,
Ölsäure und Ei-Lecithin,
Ölsäure und Ei-Kephalin,
Dimyristoylphosphatidsäure und Dimyristoyllecithin,
Dipalmitoylphosphatidsäure und 1-Palmitoyl-2-oleoyllecithin,
1-Palmitoyl-2-oleoylphosphatidsäure und Dipalmitoyllecithin,
1-Palmitoyl-2-oleoylphosphatidsäure und 1-Palmitoyl-2-oleoyllecithin,
Ei-Lysophosphatidsäure und Ei-Lecithin,
1-Myristoyllysophosphatidsäure und 1-Palmitoyl-2-oleoyllecithin,
1-Palmitoyllysophosphatidsäure und 1-Palmitoyl-2-oleoyllecithin,
Lysophosphatidylserin aus dem Rinderhirn und Ei-Lecithin,
1-Palmitoyl-lysophosphatidylserin,
1-Palmitoyl-2-oleoyl-phosphatidylserin und 1-Palmitoyl-2-oleoyllecithin und Lysophosphatidylserin aus dem Rinderhirn und Ei-Kephalin

herstellen.

Beispiel 18:

2 mg Hydrocortison-21-palmitat, 40 g Ei-Lecithin und 20 mg Ei-phosphatidsäure werden in 5 ml tert.-Butanol gelöst, durch ein 0,2 µm-Filter sterilfiltriert, in eine 25 ml Viale gefüllt, durch Eintauchen der Viale in eine Trockeneis/Äthanol-Kältemischung gefroren und lyophylisiert. Man versetzt den entstandenen Schaum mit 5 ml sterilem destilliertem Wasser und dispergiert durch 10 Minuten langes Schütteln. Durch Zugabe von 0,1 N sterilfiltrierter Natronlauge bringt man den pH-Wert auf 10,5 und lässt eine Minute lang stehen. Anschliessend setzt man 0,5 ml eines 10-fachen Konzentrates von phosphatgepufferter isotonischer Kochsalzlösung von pH 7,4 (PBS für Injektionszwecke) zu. Die so erhaltene Dispersion von unilamellaren Liposomen eignet sich zur Injektion in entzündlich veränderten Gelenkkapseln.

Beispiel 19:
0,1 mg
N-Acetylmuramyl-L-alanyl-D-isoglutamyl-L-alanyl-2-(1′,2′-dipalmitoyl-sn-glycero-3′-phosphoryl)-äthylamid,
7 mg chromatographisch gereinigtes Lecithin aus dem Hühnereiweiss und 3 mg Ei-Phosphatidsäure werden in 2 ml einer Chloroform/Methanol Mischung (2:1) gelöst und im Vakuum eingedampft. Es bleibt ein klarer Lipidfilm zurück. Man dispergiert diesen Film durch Zugabe von 2 ml sterilem destilliertem Wasser unter Umschwenken und setzt einen Tropfen 0,1%-iger Thymolphthalein-Lösung hinzu. Zur Dispersion gibt man bis zum Farbumschlag 0,1 N Natronlauge, worauf spontane Bildung von unilamellaren Liposomen erfolgt. Man puffert anschiessend sofort durch Zugabe von 0,2 ml eines 10-fachen Konzentrats von phosphatgepufferter isotonischer Kochsalzlösung (PBS für Injektionszwecke) den pH-Wert auf 7,4 ab.

Die entstandene Dispersion eignet sich direkt zur Aktivierung von alveolären Makrophagen in Zellkulturen oder in vivo in der Ratte.

Beispiel 20:
0,15 g
N-Acetylmuramyl-L-alanyl-D-isoglutamyl-L-alanyl-
2-(1′,2′-dipalmitoyl-sn-glycero-3′-phosphoryl)-äthylamid,
27 g Ei-Lecithin mit 97% Phosphatidylcholingehalt und 3 g Ei-Phosphatidsäure werden in einer Mischung von 200 ml Chloroform und 20 ml Methanol gelöst, mit 200 ml tert.-Butanol aufgefüllt und auf 180 ml eingeengt. Die Lösung wird mit einem 0,2 µm Filter sterilfiltriert, in einer Äthanol/Trockeneis Mischung rasch gefroren und anschliessend gefriergetrocknet. Das durch Mahlung zerkleinerte Lyophilisat wird in 300 ml steril hergestellte 0,01 N Natriumhydroxid-Lösung unter kräftigem Rühren eingetragen. Nach vollständiger Dispersion wird die wässrige Phase durch Zugabe von 0,1 n HCl neutralisiert. Die erhaltene opaleszierende Dispersion wird in eine gerührte Ultrafiltrationszelle (Amicon®), eingefüllt, die anstelle des Ultrafilters mit einem geradporigen Filter aus Polycarbonat (Nucelopore®) mit einem Porendurchmesser von 0,1 µm versehen ist und partikelfrei gewaschen wurde, und unter geringem Überdruck und stetiger Zufuhr von sterilfiltrierter Pufferlösung nach Dulbecco (pH 7,4 ohne Ca und Mg) so filtriert, dass das Volumen in der Zelle nicht unter 300 ml sinkt. Nach Durchtritt von 3 l Filtrat sind alle GUL abgetrennt und die überstehende Dispersion an GUL kann ampulliert und für Behandlungsversuche eingesetzt werden.

Beispiel 21:
15 mg
N-Acetylmuramyl-L-alanyl-D-isoglutamyl-L-alanyl-
2-(1′,2′-dipalmitoyl-sn-glycero-3′-phosphoryl)-äthylamid,
0,6 g reines Ei-Lecithin und 2,4 g Ei-Phosphatidsäure werden in einer Mischung von 20 ml Chloroform und 2 ml Methanol gelöst, durch ein 0,2 µm-Filter sterilfiltriert und an einem partikelfrei gewaschenen, über Sterilfilter entlüfteten Rotationsverdampfer in einem 500 ml Rundkolben so eingedampft, dass die Lipidmischung als möglichst gleichmässiger Film auf der Kolbenwand trocknet. Nach Trocknung des Rückstands über Nacht im Hochvakuum werden 30 ml steril hergestellte 0,01 N Natriumhydroxid-Lösung zugegeben, der Kolben verschlossen und 5 Minuten lang geschüttelt. Die entstandene opaleszierende wässrige Phase wird durch Zugabe von steriler 0,1 N Salzsäure auf pH 7,4 gestellt. Nach Einfüllen in eine gerührte Filterzelle (Totalvolumen 100 ml) gemäss Beispiel 20 wird unter Zugabe von sterilem, partikelfrei filtriertem Wasser so lange filtriert, bis 500 ml Filtrat gesammelt sind. Dieses Filtrat wird in eine gerührte Filterzelle, die mit einem Ultrafilter, z.B. Amicon U 10®, bestückt ist, kontinuierlich eingespeist und auf ein Volumen von 30 ml konzentriert. Die konzentrierte Dispersion enthält kleine, unilamellare Liposomen und kann nach Zugabe eines Konzentrats von Phosphatpuffer nach Dulbecco (pH 7,4 ohne Ca und Mg) ampulliert und für Behandlungsversuche eingesetzt werden.

**Patentansprüche**

1. Verfahren zur Herstellung von unilamellaren Liposomen, dadurch gekennzeichnet, dass man
a) ein Lipid der Formel

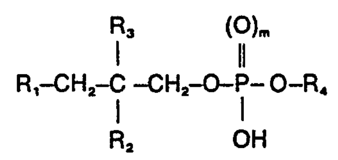

(A),

worin m null oder eins ist, einer der Reste $R_1$ und $R_2$ Wasserstoff, Hydroxy, $C_1$–$C_4$-Alkyl und der andere Rest Alkyl, Alkenyl, Alkoxy oder Alkenyloxy mit je 10–20 C-Atomen oder Acyloxy mit 10–50 C-Atomen, $R_3$ Wasserstoff oder $C_1$–$C_4$-Alkyl und $R_4$ Wasserstoff, $C_1$–$C_4$-Alkyl, $C_1$–$C_7$-Alkyl substituiert durch Carboxy oder Sulfo, $C_1$–$C_7$-Alkyl substituiert durch Carboxy und Amino, wobei die Aminogruppe sich in α-Stellung zur Carboxygruppe befindet, $C_1$–$C_4$-Alkyl substituiert durch Halogen, $C_1$–$C_4$-Alkoxycarbonyl oder $C_1$–$C_4$-Alkansulfonyl oder $C_2$–$C_4$-Alkyl substituiert durch freie oder verätherte Hydroxygruppen, wobei zwei verätherte Hydroxygruppen durch Methylen, Äthylen, Äthyliden,

1,2-Propylen oder 2,2-Propylen miteinander verbunden sein können, einen Kohlehydratrest mit 5–12 C-Atomen oder, wenn $R_1$ und $R_2$ Wasserstoff oder Hydroxy- und $R_3$ Wasserstoff bedeuten, einen Steroidrest bedeuten, und ein zusätzliches Lipid der Formel A, worin m null oder eins ist, $R_1$ und $R_2$ unabhängig voneinander Alkyl, Alkenyl, Alkoxy oder Alkenyloxy mit je 10–20 C-Atomen oder Acyloxy mit 10–50 C-Atomen, $R_3$ Wasserstoff und $R_4$ durch Tri-$C_1$–$C_4$-alkylammonio substituiertes $C_2$–$C_4$-Alkyl oder 2-Aminoäthyl bedeuten,
oder dass man ein Lipid der Formel A, worin m null oder eins ist, $R_1$ und $R_2$ unabhängig voneinander Alkyl, Alkenyl, Alkoxy mit je 10–20 C-Atomen oder Acyloxy mit 10–50 C-Atomen, $R_3$ und $R_4$ Wasserstoff bedeuten und gegebenenfalls eines der genannten zusätzlichen Lipide der Formel A in wässriger Phase mit einem pH-Wert grösser als neun dispergiert, oder dass man

b) ein Lipid der Formel A, worin m null oder eins ist, einer der Reste $R_1$ und $R_2$ Wasserstoff, Hydroxy, $C_1$–$C_4$-Alkyl und der andere Rest Alkyl, Alkenyl, Alkoxy oder Alkenyloxy mit je 10–20 C-Atomen oder Acyloxy mit 10–50 C-Atomen, $R_3$ Wasserstoff und $R_4$ durch eine Ammoniogruppe substituiertes $C_2$–$C_4$-Alkyl bedeuten, und gegebenenfalls eins der unter a) genannten zusätzlichen Lipide der Formel A oder dass man

ein Lipid der Formel A, worin m null oder eins ist, $R_1$ und $R_2$ unabhängig voneinander Alkyl, Alkenyl oder Alkenyl oder Alkenyloxy mit je 10–20 C-Atomen oder Acyloxy mit 10–50 C-Atomen, $R_3$ Wasserstoff und $R_4$ durch eine Ammonio-$C_2$–$C_4$-alkylammoniogruppe substituiertes $C_2$–$C_4$-Alkyl bedeuten, und eins der unter a) genannten zusätzlichen Lipide der Formel A in wässriger Phase mit einem pH-Wert bis ca. 1 oder darunter dispergiert und die wässrige Phase neutralisiert und, wenn erwünscht, die erhältlichen unilamellaren Liposomen anreichert und/oder abtrennt.

2. Verfahren nach Anspruch 1, dadurch gekennzeichnet, dass die wässrige Dispersion ein Lipid der Formel A, worin m eins ist, $R_1$ $C_{10}$–$C_{20}$-Alkyl, $C_{10}$–$C_{20}$-Alkoxy, $C_{12}$–$C_{20}$-Acyloxy, $R_2$ Wasserstoff oder Hydroxy, $R_3$ Wasserstoff oder Methyl und $R_4$ Wasserstoff, $C_1$–$C_4$-Alkyl
2-Amino-2-carboxyäthyl oder
3-Amino-3-carboxy-n-propyl, 2-Hydroxyäthyl,
2,3-Hydroxypropyl, 2,3-Äthylendioxypropyl,
2,3-(2,2-Propylen)-dioxypropyl,
Halogen-$C_2$–$C_4$-alkyl
oder einen Kohlehydratrest mit 5–12 C-Atomen bedeuten, und ein zusätzliches Lipid der Formel A, worin $R_1$ und $R_2$ $C_{10}$–$C_{20}$-Acyloxy, $R_3$ Wasserstoff und $R_4$ 2-Trimethylammonioäthyl oder 2-Aminoäthyl bedeuten, enthält.

3. Verfahren nach Anspruch 1, dadurch gekennzeichnet, dass die wässrige Dispersion ein Lipid der Formel A, worin $R_1$ und $R_2$ $C_{10}$–$C_{20}$-Alkanoyloxy, $R_3$ und $R_4$ Wasserstoff bedeuten, und gegebenenfalls ein zusätzliches Lipid der Formel A, worin $R_1$ und $R_2$ $C_{10}$-$C_{20}$-Alkanoyloxy, $R_3$ Wasserstoff und $R_4$ 2-Trimethylammonioäthyl, 2-Aminoäthyl, 2-Amino-2-carboxyäthyl, 3-Amino-3-carboxy-n-propyl oder den Inositolrest bedeuten, enthält.

4. Verfahren nach Anspruch 1, dadurch gekennzeichnet, dass die wässrige Dispersion natürliche Lysophosphatidsäure, synthetische Lysophosphatidsäure, natürliches Lysophosphatidylserin, synthetisches Lysophosphatidylserin, Lysophosphatidylglycerin oder Lysophosphatidylinositol und zusätzlich natürliches Lecithin, synthetisches Lecithin mit gleichen $C_{12}$–$C_{20}$-Alkanoyloxy- oder $C_{12}$–$C_{20}$-Alkenoyloxygruppen, synthetisches Lecithin mit verschiedenen $C_{12}$–$C_{20}$-Alkanoyloxy- oder $C_{12}$–$C_{20}$-Alkenoyloxygruppen, natürliches Kephalin, synthetisches Kephalin mit gleichen $C_{12}$–$C_{20}$-Alkanoyloxy- oder $C_{12}$–$C_{20}$-Alkenoyloxygruppen oder synthetisches Kephalin mit verschiedenen $C_{12}$–$C_{20}$-Alkanoyloxy- oder $C_{12}$–$C_{20}$-Alkenoyloxygruppen enthält.

5. Verfahren nach Anspruch 1, dadurch gekennzeichnet, dass die wässrige Dispersion natürliche Phosphatidsäure, synthetische Phosphatidsäure und gegebenenfalls zusätzlich natürliches Lecithin, synthetisches Lecithin mit gleichen $C_{12}$–$C_{20}$-Alkanoyloxy- oder $C_{12}$–$C_{20}$-Alkenoyloxygruppen, synthetisches Lecithin mit verschiedenen $C_{12}$–$C_{20}$-Alkanoyloxy- oder $C_{12}$–$C_{20}$-Alkenoyloxygruppen, natürliches Kephalin, synthetisches Kephalin mit gleichen $C_{12}$–$C_{20}$-Alkanoyloxy- oder $C_{12}$–$C_{20}$-Alkenoyloxygruppen oder synthetisches Kephalin mit verschiedenen $C_{12}$–$C_{20}$-Alkanoyloxy- oder $C_{12}$–$C_{20}$-Alkenoyloxygruppen enthält.

6. Verfahren nach Anspruch 1, dadurch gekennzeichnet, dass die wässrige Dispersion Ei-Phosphatidsäure oder Ei-Phosphatidsäure und Ei-Lecithin enthält.

7. Verfahren nach Anspruch 1, dadurch gekennzeichnet, dass die wässrige Dispersion Ei-Phosphatidsäure, Ei-Lecithin oder Phosphatidylserin aus dem Rinderhirn enthält.

8. Verfahren nach Anspruch 1, dadurch gekennzeichnet, dass die wässrige Dispersion Asolectin enthält.

9. Verfahren nach Anspruch 1, dadurch gekennzeichnet, dass die wässrige Dispersion Ei-Phosphatidsäure, Ei-Lecithin und Cholesterin enthält.

10. Verfahren nach Anspruch 1, dadurch gekennzeichnet, dass die wässrige Dispersion Lysolecithin und Ei-Lecithin enthält.

11. Verfahren nach Anspruch 1, dadurch gekennzeichnet, dass die wässrige Dispersion natürliches Lysophosphatidylserin und Ei-Lecithin enthält.

12. Verfahren nach Anspruch 1, dadurch gekennzeichnet, dass man die homogene Schicht der unter Verfahren a) genannten Lipide in wässriger Phase dispergiert und anschliessend den pH-Wert auf ca. 12 erhöht.

13. Verfahren nach Anspruch 12, dadurch gekennzeichnet, dass man den pH-Wert durch Zugabe von verdünnter wässriger Natriumhydroxid- oder Kaliumhydroxid-Lösung erhöht.

14. Verfahren nach Anspruch 1, dadurch gekennzeichnet, dass man eine homogene Schicht der unter Verfahren a) genannten Lipide in verdünnter, wässriger Natriumhydroxid- oder Kaliumhydroxid-Lösung dispergiert.

15. Verfahren nach Anspruch 1, dadurch gekennzeichnet, dass man die homogene Schicht der unter Verfahren b) genannten Lipide in wässriger Phase mit einem pH von ca. 1 dispergiert.

16. Verfahren nach Anspruch 1, dadurch gekennzeichnet, dass man die gemäss a) herstellbare wässrige Phase durch Zugabe von physiologisch annehmbaren Säuren oder von Pufferlösung mit einem pH 7–8 neutralisiert.

17. Verfahren nach Anspruch 16, dadurch gekennzeichnet, dass man die wässrige Phase durch Zugabe von Salzsäure neutralisiert.

18. Verfahren nach Anspruch 1, dadurch gekennzeichnet, dass man die gemäss b) herstellbare wässrige Phase durch Zugabe von physiologisch annehmbaren Basen neutralisiert.

**Claims**

1. A process for the preparation of unilamellar liposomes, characterised in that
a) a lipid of the formula

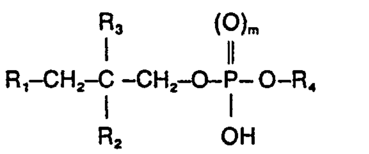

(A),

wherein m is 0 or 1, one of the radicals $R_1$ and $R_2$ is hydrogen, hydroxy or $C_1$–$C_4$alkyl, and the other is alkyl, alkenyl, alkoxy or alkenyloxy, each of from 10 to 20 carbon atoms, or is acyloxy of from 10 to 50 carbon atoms, $R_3$ is hydrogen or $C_1$–$C_4$alkyl, and $R_4$ is hydrogen, $C_1$–$C_4$alkyl, $C_1$–$C_7$alkyl substituted by carboxyl or by sulfo, $C_1$–$C_7$alkyl substituted by carboxyl and by amino, the amino group being in the α-position to the carboxyl group, $C_1$–$C_4$alkyl substituted by halogen, $C_1$–$C_4$-alkoxycarbonyl or by $C_1$–$C_4$alkanesulfonyl, or $C_2$–$C_4$alkyl substituted by free or etherified hydroxy groups, where two etherified hydroxy groups may be linked to each other through methylene, ethylene, ethylidene, 1,2-propylene or 2,2-propylene, or is a carbohydrate radical of from 5 to 12 carbon atoms, or, if $R_1$ and $R_2$ are hydrogen or hydroxy and $R_3$ is hydrogen, is a steroid radical, and an additional lipid of the formula A, wherein m is 0 or 1, each of $R_1$ and $R_2$ independently of the other is alkyl, alkenyl, alkoxy or alkenyloxy, each of from 10 to 20 carbon atoms, or is acyloxy of from 10 to 50 carbon atoms, $R_3$ is hydrogen, and $R_4$ is $C_2$–$C_4$alkyl substituted by tri-$C_1$–$C_4$alkylammonio, or is 2-aminoethyl, or a lipid of the formula A wherein m is 0 or 1, each of $R_1$ and $R_2$ independently of the other is alkyl, alkenyl, alkoxy, each of from 10 to 20 carbon atoms, or is acyloxy of from 10 to 50 carbon atoms, $R_3$ and $R_4$ are hydrogen, and optionally one of the mentioned additional lipids of the formula A, is dispersed in an aqueous phase having a pH value higher than 9, or

b) a lipid of the formula A, wherein m is 0 or 1, one of the radicals $R_1$ and $R_2$ is hydrogen, hydroxy or $C_1$–$C_4$alkyl, and the other is alkyl, alkenyl, alkoxy or alkenyloxy, each of from 10 to 20 carbon atoms, or is acyloxy of from 10 to 50 carbon atoms, $R_3$ is hydrogen, and $R_4$ is $C_2$–$C_4$alkyl which is substituted by an ammonio group, and optionally one of the additional lipids of the formula A as specified under a); or

a lipid of the formula A, wherein m is 0 or 1, each of $R_1$ and $R_2$ independently of the other is alkyl, alkenyl or alkenyl or alkenyloxy, each of from 10 to 20 carbon atoms, or is acyloxy of from 10 to 50 carbon atoms, $R_3$ is hydrogen, and $R_4$ is $C_2$–$C_4$alkyl which is substituted by an ammonio-$C_2$–$C_4$alkylammonio group, and one of the additional lipids of the formula A as specified under a) is despersed in an aqueous phase having a pH value of about 1 or below, and the aqueous phase is neutralised, and, if desired, the unilamellar liposomes so obtained are enriched and/or separated.

2. A process according to claim 1, charaterised in that the aqueous dispersion contains a lipid of the formala A, wherein m is 1, $R_1$ is $C_{10}$–$C_{20}$alkyl, $C_{10}$–$C_{20}$alkoxy, or $C_{10}$–$C_{20}$acyloxy, $R_2$ is hydrogen or hydroxy, $R_3$ is hydrogen or methyl, and $R_4$ is hydrogen, $C_1$–$C_4$alkyl,
2-amino-2-carboxyethyl or
3-amino-3-carboxy-n-propyl,
2-hydroxyethyl, 2,3-hydroxypropyl,
2,3-ethylenedioxypropyl,
2,3-(2,2-propylene)dioxypropyl,
halo-$C_2$–$C_4$alky,
or a carbohydrate radical of from 5 to 12 carbon atoms, and an additional lipid of the formula A, wherein $R_1$ and $R_2$ are $C_{10}$–$C_{20}$acyloxy, $R_3$ is hydrogen and $R_4$ is 2-trimethylammonioethyl or 2-aminoethyl.

3. A process according to claim 1, characterised in that the aqueous dispersion contains a lipid of the formula A, wherein $R_1$ and $R_2$ are $C_{10}$–$C_{20}$alkanoyloxy, and $R_3$ and $R_4$ are hydrogen, and optionally an additional lipid of the formula A, wherein $R_1$ and $R_2$ are $C_{10}$–$C_{20}$alkanoyloxy, $R_3$ is hydrogen and $R_4$ is 2-trimethylammonioethyl, 2-aminoethyl, 2-amino-2-carboxyethyl, 3-amino-3-carboxy-n-propyl, or the inositol radical.

4. A process according to claim 1, characterised in that the aqueous dispersion contains a natural lysophosphatidic acid, a synthetic lysophosphatidic acid, a natural lysophosphatidylserine, a synthetic lysophosphatidylserine, a lysophosphatidylglycerol or a lysophosphatidylinositol, and additionally a natural lecithin, a synthetic lecithin containing identical $C_{12}$–$C_{20}$alkanoyloxy or $C_{12}$–$C_{20}$alkenoyloxy groups, a synthetic lecithin containing different $C_{12}$–$C_{20}$alkanoyloxy or $C_{12}$–$C_{20}$alkenoyloxy groups, a natural cephalin, a synthetic cephalin containing identical $C_{12}$–$C_{20}$alkanoyloxy or $C_{12}$–$C_{20}$alkenoyloxy groups or a synthetic cephalin containing different $C_{12}$–$C_{20}$alkanoyloxy or $C_{12}$–$C_{20}$alkenoyloxy groups.

5. A process according to claim 1, characterised in that the aqueous dispersion contains a natural phosphatidic acid, a synthetic phosphatidic acid, and optionally additionally a natural lecithin, a synthetic lecithin containing identical $C_{12}$–$C_{20}$alkanoyloxy or $C_{12}$–$C_{20}$alkenoyloxy groups, a synthetic lecithin containing different $C_{12}$–$C_{20}$alkanoyloxy or $C_{12}$–$C_{20}$alkenoyloxy groups, a natural ce-

phalin, a synthetic cephalin containing identical $C_{12}$–$C_{20}$alkanoyloxy or $C_{12}$–$C_{20}$alkenoyloxy groups, or a synthetic cephalin containing different $C_{12}$–$C_{20}$alkanoyloxy or $C_{12}$–$C_{20}$alkenoyloxy groups.

6. A process according to claim 1, characterised in that the aqueous dispersion contains egg phosphatidic acid, or egg phosphatidic acid and egg lecithin.

7. A process according to claim 1, characterised in that the aqueous dispersion contains egg phosphatidic acid, egg lecithin or beef brain phosphatidylserine.

8. A process according to claim 1, characterised in that the aqueous dispersion contains asolectin.

9. A process according to claim 1, characterised in that the aqueous dispersion contains egg phosphatidic acid, egg lecithin and cholesterol.

10. A process according to claim 1, characterised in that the aqueous dispersion contains lysolecithin and egg lecithin.

11. A process according to claim 1, characterised in that the aqueous dispersion contains natural lysophosphatidylserine and egg lecithin.

12. A process according to claim 1, characterised in that a homogeneous layer of the lipids as specified under process a) is dispersed in an aqueous phase and the pH value is subsequently raised to about 12.

13. A process according to claim 12, characterised in that the pH value is raised by the addition of dilute aqueous sodium hydroxide solution or dilute aqueous potassium hydroxide solution.

14. A process according to claim 1, characterised in that a homogeneous layer of the lipids as specified under process a) is dispersed in dilute aqueous sodium hydroxide solution or dilute aqueous potassium hydroxide solution.

15. A process according to claim 1, characterised in that a homogeneous layer of the lipids as specified under process b) is dispersed in an aqueous phase having a pH of about 1.

16. A process according to claim 1, characterised in that the aqueous phase that can be prepared in accordance with a) is neutralised by the addition of a physiologically acceptable acid, or a buffer solution having a pH of from 7 to 8.

17. A process according to claim 16, characterised in that the aqueous phase is neutralised by the addition of hydrochloric acid.

18. A process according to claim 1, characterised in that the aqueous phase that can be prepared in accordance with b) is neutralised by the addition of a physiologically acceptable base.

**Revendications**

1. Procédé de préparation de liposomes unilamellaires caractérisé en ce que:

a) on disperse un lipide de formule

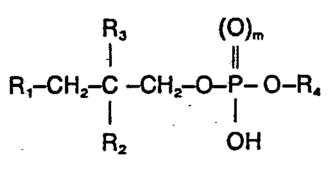 (A),

dans laquelle m est égal à 0 ou 1, l'un des symboles $R_1$ ou $R_2$ représente l'hydrogène, un groupe hydroxy, alkyle en C 1–C 4 et l'autre un groupe alkyle, alcényle, alcoxy ou alcényloxy contenant chacun 10 à 20 atomes de carbone ou acyloxy contenant 10 à 50 atomes de carbone, $R_3$ représente l'hydrogène ou un groupe alkyle en C 1–C 4 et $R_4$ représente l'hydrogène, un groupe alkyle en C 1–C 4, alkyle en C 1–C 7 substitué par un groupe carboxy ou sulfo, alkyle en C 1–C 7 substitué par un groupe carboxy et un groupe amino, le groupe amino se trouvant en position alpha du groupe carboxy, alkyle en C 1–C 4 substitué par un halogène, un groupe (alcoxy en C 1–C 4)-carbonyle ou alcane-sulfonyle en C 1–C 4 ou un groupe alkyle en C 2–C 4 substitué par des groupes hydroxy libres ou éthérifiés, deux groupes hydroxy éthérifiés pouvant être reliés entre eux par un groupe méthylène, éthylène, éthylidène, 1,2-propylène ou 2,2-propylène, un reste d'hydrate de carbone en C 5–C 12 ou bien encore, lorsque $R_1$ et $R_2$ représentent l'hydrogène ou des groupes hydroxy et $R_3$ l'hydrogène, un reste de stéroïde, et un lipide supplémentaire de formule A dans laquelle m est égal à 0 ou 1, $R_1$ et $R_2$ représentent chacun, indépendamment l'un de l'autre, un groupe alkyle, alcényle, alcoxy ou alcényloxy contenant chacun 10 à 20 atomes de carbone ou acyloxy contenant 10 à 50 atomes de carbone, $R_3$ représente l'hydrogène et $R_4$ un groupe alkyle en C 2–C 4 substitué par un groupe tri-(alkyle en C 1–C 4)-ammonio ou un groupe 2-aminoéthyle,

ou bien on disperse un lipide de formule A dans laquelle m est égal à 0 ou 1, $R_1$ et $R_2$ représentent chacun, indépendamment l'un de l'autre, un groupe alkyle, alcényle, alcoxy contenant chacun 10 à 20 atomes de carbone ou acyloxy contenant 10 à 50 atomes de carbone, $R_3$ et $R_4$ représentent l'hydrogène et, le cas échéant, l'un des lipides supplémentaires de formule A mentionnés ci-dessus en phase aqueuse à un pH supérieur à 9, ou bien b) on disperse un lipide de formula A dans laquelle m est égal à 0 ou 1, l'un des symboles $R_1$ et $R_2$ représente l'hydrogène, un groupe hydroxy, alkyle en C 1–C 4 et l'autre un groupe alkyle, alcényle, alcoxy ou alcényloxy contenant chacun 10 à 20 atomes de carbone ou acyloxy contenant 10 à 50 atomes de carbone, $R_3$ représente l'hydrogène et $R_4$ un groupe alkyle en C 2–C 4 substitué par un groupe ammonio, et le cas échéant, l'un des lipides supplémentaires de formula A mentionnès sous a), ou bien

on disperse un lipide de formule A dans laquelle m est égal à 0 ou 1, $R_1$ et $R_2$ représentent chacun, indépendamment l'un de l'autre, un groupe alkyle, alcényle ou alcényle ou alcényloxy contenant chacun 10 à 20 atomes de carbone ou acyloxy contenant 10 à 50 atomes de carbone, $R_3$ représente l'hydrogène et $R_4$ un groupe alkyle en C 2–C 4 substitué par un groupe ammonio-(alkyle en C 2–C 4)-ammonio, et l'un des lipides supplémentaires de formula A mentionnés sous a) en phase aqueuse à un pH de 1 environ ou au-dessous et on neutralise la phase aqueuse, et, si

on le désire, on enrichit et/ou on sépare les liposomes unilamellaires obtenus.

2. Procédé selon la revendication 1, caractérisé en ce que la dispersion aqueuse contient un lipide de formule A dans laquelle m est égal à 1, $R_1$ représente un groupe alkyle en C 10–C 20, alcoxy en C 10–C 20, alcyloxy en C 10–C 20, $R_2$ représente l'hydrogène ou un groupe hydroxy, $R_3$ représente l'hydrogène ou un groupe méthyle et $R_4$ représente l'hydrogène, un groupe alkyle en C 1–C 4, 2-amino-2-carboxyéthyle ou 3-amino-3-carboxy-n-propyle, 2-hydroxyéthyle, 2,3-hydroxypropyle, 2,3-éthylène-dioxypropyle, 2,3-(2,2-propylène)-dioxypropyle, halogénoalkyle en C 2–C 4 ou un reste d'hydrate de carbone en C 5–C 12, et un lipide supplémentaire de formule A dans laquelle $R_1$ et $R_2$ représentent des groupes acyloxy en C 10–C 20, $R_3$ représente l'hydrogène et $R_4$ un groupe 2-triméthylammonioéthyle ou 2-aminoéthyle.

3. Procédé selon la revendication 1, caractérisé en ce que la dispersion aqueuse contient un lipide de formule A dans laquelle $R_1$ et $R_2$ représentent des groupes alcanoyloxy en C 10–C 20, $R_3$ et $R_4$ représentent l'hydrogène et, le cas échéant, un lipide supplémentaire de formule A dans laquelle $R_1$ et $R_2$ représentent des groupes alcanoyloxy en C 10–C 20, $R_3$ représente l'hydrogène et $R_4$ représente un groupe 2-triméthylammonioéthyle, 2-aminoéthyle, 2-amino-2-carboxyéthyle, 3-amino-3-carboxy-n-propyle ou le reste de l'inositol.

4. Procédé selon la revendication 1, caractérisé en ce que la dispersion aqueuse contient de l'acide lysophosphatidique naturel, de l'acide lysophosphatidique synthetique, une lysophosphatidylsérine naturelle, une lysophosphatidylsérine synthétique, du lysophosphatidylglycérol ou du lysophosphatidylinositol et en plus de la lécithine naturelle, une lécithine synthétique contenant des groupes alcanoyloxy en C 12–C 20 ou alcénoyloxy en C 12–C 20 identiques, une lécithine synthétique contenant des groupes alcanoyloxy en C 12–C 20 ou alcénoyloxy en C 12–C 20 différents, une céphaline naturelle, une céphaline synthétique contenant des groupes alcanoyloxy en C 12–C 20 ou alcénoyloxy en C 12–C 20 identiques ou une céphaline synthétique contenant des groupes alcanoyloxy en C 12– C 20 ou alcénoyloxy en C 12–C 20 différents.

5. Procédé selon la revendication 1, caractérisé en ce que la dispersion aqueuse contient de l'acide phosphatidique naturel, de l'acide phosphatidique synthétique et, le cas échéant, en outre de la lécithine naturelle, de la lécithine synthétique à groupes alcanoyloxy en C 12–C 20 ou alcénoyloxy en C 12–C 20 identiques, une lécithine synthétique à groupes alcanoyloxy en C 12–C 20 ou alcénoyloxy en C 12–C 20 différents, une céphaline naturelle, une céphaline synthétique à groupes alcanoyloxy en C 12– C 20 ou alcénoyloxy en C 12–C 20 identiques, ou une céphaline synthétique à groupes alcanoyloxy en C 12–C 20 ou alcénoyloxy en C 12–C 20 différents.

6. Procédé selon la revendication 1, caractérisé en ce que la dispersion aqueuse contient de l'acide phosphatidique d'œuf ou de l'acide phosphatidique d'œuf et de la lécithine d'œuf.

7. Procédé selon la revendication 1, caractérisé en ce que la dispersion aqueuse contient de l'acide phosphatidique d'œuf, de la lécithine d'œuf ou de la phosphatidylsérine de cervelle de bovin.

8. Procédé selon la revendication 1, caractérisé en ce que la dispersion aqueuse contient de l'asolectine.

9. Procédé selon la revendication 1, caractérisé en ce que la dispersion aqueuse contient de l'acide phosphatidique d'œuf, de la lécithine d'œuf et du cholestérol.

10. Procédé selon la revendication 1, caractérisé en ce que la dispersion aqueuse contient de la lysolécithine et de la lécithine d'œuf.

11. Procédé selon la revendication 1, caractérisé en ce que la dispersion aqueuse contient de la lysophosphatidylsérine naturelle et de la lécithine d'œuf.

12. Procédé selon la revendication 1, caractérisé en ce que l'on disperse la couche homogène des lipides mentionnés en référence au procédé a) en phase aqueuse et on porte ensuite le pH à 12 environ.

13. Procédé selon la revendication 1, caractérisé en ce que l'on accroît le pH par addition d'une solution aqueuse diluée d'hydroxyde de sodium ou d'hydroxyde de potassium.

14. Procédé selon la revendication 1, caractérisé en ce que l'on disperse une couche homogène des lipides mentionnés en référence au procédé a) dans une solution aqueuse diluée d'hydroxyde de sodium ou d'hydroxyde de potassium.

15. Procédé selon la revendication 1, caractérisé en ce que l'on disperse la couche homogène des lipides mentionnés en référence au procédé b) en phase aqueuse à un pH d'environ 1.

16. Procédé selon la revendication 1, caractérisé en ce que l'on neutralise à pH 7 à 8 la phase aqueuse préparée par le procédé a) par addition d'acides acceptables pour l'usage pharmaceutique ou d'une solution tampon.

17. Procédé selon la revendication 16, caractérisé en ce que l'on neutralise la phase aqueuse par addition d'acide chlorhydrique.

18. Procédé selon la revendication 1, caractérisé en ce que l'on neutralise la phase aqueuse préparée par le procédé b) par addition de bases acceptables pour l'usage pharmaceutique.